# EUROPEAN PATENT APPLICATION

(11) **EP 1 132 477 A1**
(43) Date of publication of application: **12.09.2001**
(21) Application number: 99972687.0
(22) Date of filing: 19.11.1999
(51) Int. Cl.: C12N 15/57, C12N 9/50, C07K 14/47, C12P 21/02, A01K 67/027, C07K 16/40, G01N 33/53

(54) **NOVEL SERINE PROTEASES BSSP4**

(30) Priority: 20.11.1998 JP 34781398
(71) Applicant: FUSO PHARMACEUTICAL INDUSTRIES LTD., Osaka-shi, Osaka 541-0045 (JP)
(72) Inventor: UEMURA, Hidetoshi, Itami-shi, Hyogo 664-0883 (JP); OKUI, Akira, Yamatokoriyama-shi, Nara 639-1123 (JP); KOMINAMI, Katsuya, Hannan-shi, Osaka 599-0212 (JP); YAMAGUCHI, Nozomi, 285-79, Shingoryoguchi-cho, Kyoto-shi, Kyoto 603-8146 (JP); MITSUI, Shinichi, 202, Kitashirakawa-koporasu, Kyoto-shi, Kyoto 606-8267 (JP)
(74) Representative: VOSSIUS & PARTNER
(86) International application number: JP9906472
(87) International publication number: WO0031277

(57) **Abstract**

There are provided proteins having the amino acid sequences represented by SEQ ID NOS: 2, 4, 6, 8, 10, 12, 14, 16, 18 and 20; proteins having amino acid sequences derived from these amino acid sequences by deletion, substitution or addition of one to several amino acids; and nucleotide sequences encoding the same; transgenic non-human animals with altered expression level of a serine protease BSSP4; an antibody against BSSP4; and a method for detecting BSSP4 in a specimen by using the antibody.

## Description

### FIELD OF THE INVENTION

The present invention relates to isolated polynucleotides of human and mouse serine proteases (hereinafter referred to as "hBSSP4" and "mBSSP4", respectively, and, in case no differentiation thereof from each other is needed, simply referred to as "BSSP4"), and their homologous forms, mature forms, precursors and polymorphic variants as well as a method for detecting thereof. Further, it relates to hBSSP4 and mBSSP4 proteins, compositions containing hBSSP4 and mBSSP4 polynucleotides and proteins, as well as their production and use.

### BACKGROUND OF THE INVENTION

In general, proteases are biosynthesized as inactive precursors. They undergo limited hydrolysis in molecules to convert into activated type proteases. In so far as enzymes are proteases, they have an activity for hydrolyzing a peptide bond, while their action modes are varied according to kinds of proteases. According to a particular kind of catalytic site, proteases are divided into serine proteases, cysteine proteases, aspartate proteases, metal proteases and the like. Proteases of each kind have a variety of properties, ranging from a protease having general digestive properties to a protease having various regulatory domains and strict substrate specificity, thereby specifically hydrolyzing only characteristic proteins.

Further, proteins undergo various processing even after translation to produce active proteins. In many secretory proteins, a protein are first synthesized on the ribosome in cytoplasm as an inactive precursor (pro-form) which comprises an active protein bearing at the N-terminus thereof a peptide of about 15 to 60 amino acids responsible for secretion (secretory signal). This peptide region is concerned with the mechanism for passing through the cell membrane and is removed upon cleavage by a specific protease during the passage through the membrane, in almost all the cases, to produce a mature protein. A secretory signal has a broad hydrophobic region comprising hydrophobic amino acids in the middle of the sequence, and basic amino acid residues at a site close to the N-terminus. A secretory signal is a synonym of a signal peptide. In addition, in some proteins, a peptide moiety which functions as a secretory signal is further attached to the N-terminus of the inactive precursor (pro-form). Such a protein is called a prepro-protein (prepro-form).

For example, trypsin is present as a prepro-form immediately after translation into amino acids. After being secreted from cells, it is present as a pro-form and is converted into active trypsin in duodenum upon limited hydrolysis by enteropeptidase or by trypsin itself.

The optimal pH range of serine proteases is neutral to weak alkaline and, in general, many of them have a molecular weight of about 30,000 or lower. All proteases of blood coagulation, fibrinolysis and complement systems having a large molecular weight belong to trypsin-like serine proteases. They have many regulator domains and form a protease cascade which is of very importance to reactions in a living body.

Recently, cDNAs and amino acid sequences of many novel proteases have been determined by PCR for consensus sequences of serine proteases using oligonucleotide primers. According to this method, novel proteases have been found by various researchers such as Yamamura et al. (Yamanura, Y et al., Biochem. Biophys. Res. Commun., 239, 386, 1997), Gschwend, et al. (Gschwend, T. P. et al., Mol. Cell. Neurosci., 9. 207, 1997), Chen et al. (Chen, Z-L, et al., J. Neurosci., 15, 5088, 1995) and others.

SEQ ID NO: 3 of JP 9-149790 A discloses neurosin as a novel serine protease. Neurosin has also been reported in Biochimica et Byophysica Acta, 1350, 11-14, 1997. By this, there is provided a method for mass production of neurosin using the serine protease gene and a method for screening specific inhibitors using the enzyme. In addition, the screening method has been shown to be useful for screening medicines for treating various diseases.

Serine proteases expressed in a brain-nerve system such as neurosin are considered to play various roles in the brain-nerve system. Therefore, there is a possibility that isolation of a gene encoding a novel protease expressed in a brain-nerve system and production of a protein using the gene would be useful for diagnosis or treatment of various diseases related to the brain-nerve system.

Nowadays, in general, clinical diagnosis of Alzheimer's disease is conducted based on the diagnosis standard of DSM-IIIR and NINCDS-ADRDA (Mckhann, G. et al., Neurology, 34. 939, 1994) or the diagnosis standard of DSM-IV (American Psychiatric Association; Diagnostic and statistical manuals of mental disorders, 4th ed., Washington DC, American Psychiatric Association, 1994). However, these standards are conditioned by decline of recognition functions which causes a severe disability in a daily life or a social life. Then, it is pointed out that the diagnosis is less scientific objectivity because the diagnosis may be influenced by the level of an individual's social life and further the specialty and experience of a physician who diagnoses particular conditions. In addition, definite diagnosis of Alzheimer's disease is conducted by pathohistological analyses and, in this respect, substantial inconsistency between clinical diagnosis and autopsy diagnosis is pointed out.

At present, image diagnosis is employed as a supplemental means in clinical diagnosis of Alzheimer's diagnosis and it is possible to analyze brain functions, for example, decline of metabolism and atrophy in specific sites such as hippocampus, parietal lobe of cerebral cortex and the like which are specific for Alzheimer's disease by PET and SPECT. However, to define Alzheimer's disease based on lowering of a blood flow from parietal lobe to temporal lobe is very dangerous. In addition, there is few report showing that MRS testicle useful for patients with dementia including those of Alzheimer's disease. Further, although CT-MRI image diagnosis is used, a lesion of white matter such as atrophy of brain, PVL or the like is not specific for Alzheimer type dementia. Since it has been reported that atrophy of brain proceeds as getting older, the above observation is not necessarily found in Alzheimer type dementia. Furthermore, since an image obtained by MRI varies according to strength of a magnetic field, performance of an apparatus and imaging conditions, numerical data obtain in different facilities cannot be compared with each other except atrophic change. In addition, there is a limit to image measurement. Further, enlargement of ventricle can be recognized in vascular dementia cases and there are cases wherein atrophy of hippocampus is observed after ischemia of basilar artery.

Under these circumstances, many researchers have requested to develop biological diagnosis markers as a means for providing better precision and objectivity for clinical diagnosis of Alzheimer's disease. At the same time, the following important roles in the future will be expected.
1) Objective judgment system of effect of medicaments for treating Alzheimer's disease.
2) Detection of Alzheimer's disease before a diagnosis standard is met, or disease conditions are manifested.

Further, data obtained in different facilities can be compared with each other by using the same diagnosis marker. Therefore, development of biological diagnosis markers is recognized to be a most important field among fields of Alzheimer's disease studies and its future prospects will be expected. Approaches to development of biological diagnosis markers up to now are divided into that based on constitute components of characteristic pathological changes of Alzheimer's disease such as senile plaque and neurofibril change, and an approach based on other measures. Examples of the former include cerebrospinal fluid tau protein, Aβ and its precursor, βAPP. Examples of the latter include mydriasis test with cholilytic drug, Apo E and other genes relating to Alzheimer's disease. However, no good results are obtained.

Serine proteases are also considered to play important role in cancer cells. The reason why extermination of cancer by surgical treatment or topical irradiation of radioactive ray is difficult is metastasis capability of cancer. For spread of solid tumor cells in a body, they should loosen their adhesion to original adjacent cells, followed by separating from an original tissue, passing through other tissues to reach blood vessel or lymph node, entering into the circulatory system through stratum basal and endothelial layer of the vessel, leave from the circulatory system at somewhere in the body, and surviving and proliferating in a new environment. While adhesion to adjacent epidermal cells is lost when expression of cadherin which is an intercellular adhesive molecule of epithelium is stopped, to break through tissues is considered to depend on proteolytic enzymes which decompose an extracellular matrix.

As enzymes which decompose the matrix, mainly, metal proteases (Rha, S. Y. et al., Breast Cancer Research Treatment, 43, 175, 1997) and serine proteases are known. They cooperate to decompose matrix protein such as collagen, laminin and fibronectin. Among serine proteases known to be concerned in decomposition of the matrix, in particular, there is urokinase type plasminogen activator (U-PA). U-PA has a role as a trigger specific for a protein decomposition chain reaction. Its direct target is plasminogen. It is present in blood abundantly and is a precursor of an inactive serine protease which accumulates in reconstructed sites of tissues such as injured sites and tumors as well as inflammatory sites. In addition, as proteases which are concerned in metastasis and infiltration of cancers, for example, a tissue factor, lysosomal type hydrolase and collagenase have been known.

At present, cancer is the top cause of death in Japan and more than 200,000 people are died per year. Then, specific substances which can be used as markers for diagnosis and therapy or prophylaxis of cancer are studied intensively. Such specific substances are referred to as tumor markers or tumor marker relating biomarkers. They are utilized in aid of diagnosis before treatment of cancer, for presuming carcinogenic organ and pathological tissue type, for monitoring effect of treatment, for finding recurrence early, for presuming prognosis, and the like. At present, tumor markers are essential in clinical analyses. Among them, alpha fetoprotein (AFP) which has high specificity to hepatocellular carcinoma and yolk sac tumor (Taketa K. et al., Tumour Biol., 9, 110, 1988), and carcinoembronic antigen (CEA) are used worldwide. In the future, tumor markers will be required more and more, and it is desired to develop, for example, organ specific markers and tumor cell specific markers which are highly reliable serologic diagnosis of cancer. Up to now, humunglandular kallikrein (hK2) which is a serine protease expressed at human prostatic epithelial cells has been reported as a marker for prostatic cancer. And, hK2 has 78% homology with the sequence of prostatic specific antigen (PSA) and PSA is also used widely as a biochemical marker of prostatic cancer (Mikolajczyk, S. d. et al., Prostate, 34, 44, 1998; Pannek, J. et al., Oncology, 11, 1273, 1997; Chu, T. M. et al., Tumour Biology, 18, 123, 1997; Hsieh, M. et al., Cancer Res., 57, 2651, 1997). Further, hK2 is reported to be useful as a marker for not only prostatic cancer but also stomach cancer (Cho, J. Y. et al.. Cancer, 79, 878, 1997). Moreover, CYFRA (CYFRA 21-1) for measuring cytokeratin 19 fragment in serum is reported to be useful for lung cancer (Sugiyama, Y. et al., Japan J. Cancer Res., 85, 1178, 1994). Gastrin release peptide precursor (ProGRP) is reported to be useful as a tumor marker (Yamaguchi, K. et al., Japan, J. Cancer Res., 86, 698, 1995).

### OBJECTS OF THE INVENTION

Thus, the main object of the present invention is to provide a novel serine protease which can be used for treating or diagnosing various diseases such as Alzheimer's disease (AD), epilepsy, cancer, inflammation, sterility, prostate hypertrophy and the like in various tissues such as brain, lung, prostate, testicle, skeletal muscle, liver and the like, and can be used as an excellent marker instead of that presently used.

### SUMMARY OF THE INVENTION

Under these circumstances, the present inventors have succeeded in cloning of cDNA encoding novel human and mouse serine proteases.

In summary, one feature of the present invention is amino acid sequences of biological active mature serine proteases hBSSP4 and mBSSP4 as well as nucleotide sequences encoding the amino acid sequences.

That is, they are the amino acid sequence composed of 268 amino acids represened by the 1st to 268th amino acids of SEQ ID NO: 2 and a nucleotide sequence encoding the amino acid sequence (the 151st to 954th bases of SEQ ID NO: 1). In addition, they include amino acid sequences substantially similar to the amino acid sequence and nucleotide sequences encoding such similar amino acid sequences. Further, they include modified derivatives of proteins having these amino acid sequences. An amino acid sequence substantially similar to a given amino acid sequence used herein means an amino acid sequence derived from the given amino acid sequence by modification such as substitution, deletion, addition and/or insertion of one to several amino acids with maintaining the same property as that of the protein having the given amino acid sequence. The modified derivative of the proteins includes, for example, phosphate adduct, sugar chain adduct, metal adduct (e.g., calcium adduct), the protein fused to another protein such as albumin etc., dimer of the protein, and the like.

Further, they are the amino acid sequence composed of 270 amino acids represened by the 1st to 270th amino acids of SEQ ID NO: 4 and a nucleotide sequence encoding the amino acid sequence (the 15th to 960th bases of SEQ ID NO: 3). In addition, they include amino acid sequences substantially similar to the amino acid sequence and nucleotide sequences encoding such similar amino acid sequences. Further, they include modified derivatives of proteins having these amino acid sequences.

Further, they are the amino acid sequence composed of 257 amino acids represened by the 1st to 257th amino acids of SEQ ID NO: 6 and a nucleotide sequence encoding the amino acid sequence (the 151st to 921st bases of SEQ ID NO: 5). In addition, they include amino acid sequences substantially similar to the amino acid sequence and nucleotide sequences encoding such similar amino acid sequences. Further, they include modified derivatives of proteins having these amino acid sequences.

Further, they are the amino acid sequence composed of 97 amino acids represened by the 1st to 97th amino acids of SEQ ID NO: 8 and a nucleotide sequence encoding the amino acid sequence (the 151st to 441st bases of SEQ ID NO: 7). In addition, they include amino acid sequences substantially similar to the amino acid sequence and nucleotide sequences encoding such similar amino acid sequences. Further, they include modified derivatives of proteins having these amino acid sequences.

Further, they are the amino acid sequence composed of 158 amino acids represened by the 1st to 158th amino acids of SEQ ID NO: 10 and a nucleotide sequence encoding the amino acid sequence (the 151st to 624th bases of SEQ ID NO: 9). In addition, they include amino acid sequences substantially similar to the amino acid sequence and nucleotide sequences encoding such similar amino acid sequences. Further, they include modified derivatives of proteins having these amino acid sequences.

Further, they are the amino acid sequence composed of 82 amino acids represened by the 1st to 82nd amino acids of SEQ ID NO: 12 and a nucleotide sequence encoding the amino acid sequence (the 151st to 396th bases of SEQ ID NO: 11). In addition, they include amino acid sequences substantially similar to the amino acid sequence and nucleotide sequences encoding such similar amino acid sequences. Further, they include modified derivatives of proteins having these amino acid sequences.

Further, they are the amino acid sequence composed of 185 amino acids represened by the 1st to 185th amino acids of SEQ ID NO: 14 and a nucleotide sequence encoding the amino acid sequence (the 151st to 705th bases of SEQ ID NO: 13). In addition, they include amino acid sequences substantially similar to the amino acid sequence and nucleotide sequences encoding such similar amino acid sequences. Further, they include modified derivatives of proteins having these amino acid sequences.

Further, they are the amino acid sequence composed of 80 amino acids represened by the 1st to 80th amino acids of SEQ ID NO: 16 and a nucleotide sequence encoding the amino acid sequence (the 151st to 390th bases of SEQ ID NO: 15). In addition, they include amino acid sequences substantially similar to the amino acid sequence and nucleotide sequences encoding such similar amino acid sequences. Further, they include modified derivatives of proteins having these amino acid sequences.

Further, they are the amino acid sequence composed of 253 amino acids represened by the 1st to 253th amino acids of SEQ ID NO: 18 and a nucleotide sequence encoding the amino acid sequence (the 151st to 909th bases of SEQ ID NO: 17). In addition, they include amino acid sequences substantially similar to the amino acid sequence and nucleotide sequences encoding such similar amino acid sequences. Further, they include modified derivatives of proteins having these amino acid sequences.

Further, they are the amino acid sequence composed of 34 amino acids represened by the -49th to -16th amino acids of SEQ ID NO: 2 and a nucleotide sequence encoding the amino acid sequence (the 4th to 105th bases of SEQ ID NO: 1). In addition, they include amino acid sequences substantially similar to the amino acid sequence and nucleotide sequences encoding such similar amino acid sequences. Further, they include modified derivatives and fragments of proteins having these amino acid sequences.

Further, they are the amino acid sequence composed of 15 amino acids represened by the -15th to -1st amino acids of SEQ ID NO: 2 and a nucleotide sequence encoding the amino acid sequence (the 106th to 150th bases of SEQ ID NO: 1). In addition, they include amino acid sequences substantially similar to the amino acid sequence and nucleotide sequences encoding such similar amino acid sequences. Further, they include modified derivatives and fragments of proteins having these amino acid sequences.

Further, they are the amino acid sequence composed of 259 amino acids represened by the 1st to 259th amino acids of SEQ ID NO: 20 and a nucleotide sequence encoding the amino acid sequence (the 227th to 1003rd bases of SEQ ID NO: 19). In addition, they include amino acid sequences substantially similar to the amino acid sequence and nucleotide sequences encoding such similar amino acid sequences. Further, they include modified derivatives of proteins having these amino acid sequences.

Further, they are the amino acid sequence composed of 34 amino acids represened by the -49th to -16th amino acids of SEQ ID NO: 20 and a nucleotide sequence encoding the amino acid sequence (the 80th to 181st bases of SEQ ID NO: 19). In addition, they include amino acid sequences substantially similar to the amino acid sequence and nucleotide sequences encoding such similar amino acid sequences. Further, they include modified derivatives and fargments of proteins having these amino acid sequences.

Further, they are the amino acid sequence composed of 15 amino acids represened by the -15th to -1st amino acids of SEQ ID NO: 20 and a nucleotide sequence encoding the amino acid sequence (the 182nd to 226th bases of SEQ ID NO: 19). In addition, they include amino acid sequences substantially similar to the amino acid sequence and nucleotide sequences encoding such similar amino acid sequences. Further, they include modified derivatives and fragements of proteins having these amino acid sequences.

Another feature of the present invention is an amino acid sequence composed of 317 or 283 amino acids wherein 49 amino acids represented by the -49th to -1st amino acids or 15 amino acids represented by the -15th to -1st amino acids of SEQ ID NO: 2 are added to the N-terminus side of the mature hBSSP4 amino acid sequence represented by SEQ ID NO: 2 (the 1st to 268th amino acids) and a nucleotide sequence encoding the amino acid sequence (the 4th to 954th or 106th to 954th bases of SEQ ID NO: 1). In addition, this feature includes amino acid sequences substantially similar to the above amino acid sequence and nucleotide sequences encoding these substantially similar amino acid sequences. Further, this feature includes modified derivatives of proteins having these amino acid sequences.

Another feature of the present invention is an amino acid sequence composed of 319 or 285 amino acids wherein 49 amino acids represented by the -49th to -1st amino acids or 15 amino acids represented by the -15th to -1st amino acids of SEQ ID NO: 4 are added to the N-terminus side of the mature hBSSP4 amino acid sequence represented by SEQ ID NO: 4 (the 1st to 270th amino acids) and a nucleotide sequence encoding the amino acid sequence (the 4th to 960th or 106th to 960th bases of SEQ ID NO: 3). In addition, this feature includes amino acid sequences substantially similar to the above amino acid sequence and nucleotide sequences encoding these substantially similar amino acid sequences. Further, this feature includes modified derivatives of proteins having these amino acid sequences.

Another feature of the present invention is an amino acid sequence composed of 306 or 272 amino acids wherein 49 amino acids represented by the -49th to -1st amino acids or 15 amino acids represented by the -15th to -1st amino acids of SEQ ID NO: 6 are added to the N-terminus side of the mature hBSSP4 amino acid sequence represented by SEQ ID NO: 6 (the 1st to 257th amino acids) and a nucleotide sequence encoding the amino acid sequence (the 4th to 921st or 106th to 921st bases of SEQ ID NO: 5). In addition, this feature includes amino acid sequences substantially similar to the above amino acid sequence and nucleotide sequences encoding these substantially similar amino acid sequences. Further, this feature includes modified derivatives of proteins having these amino acid sequences.

Another feature of the present invention is an amino acid sequence composed of 308 or 274 amino acids wherein 49 amino acids represented by the -49th to -1st amino acids or 15 amino acids represented by the -15th to -1st amino acids of SEQ ID NO: 20 are added to the N-terminus side of the mature mBSSP4 amino acid sequence represented by SEQ ID NO: 20 (the 1st to 259th amino acids) and a nucleotide sequence encoding the amino acid sequence (the 8th to 1003rd or 182nd to 1003rd bases of SEQ ID NO: 19). In addition, this feature includes amino acid sequences substantially similar to the above amino acid sequence and nucleotide sequences encoding these substantially similar amino acid sequences. Further, this feature includes modified derivatives of proteins having these amino acid sequences.

The present invention also relates to the nucleotide sequences represented by SEQ ID NOS: 1, 3, 5, 7, 9, 11, 13, 15, 17 and 19 as well as nucleotide sequences similar to them.

Hereinafter, unless otherwise stated, the nucleotide sequence represented by each SEQ ID NO: includes the above-described various fragments thereof, and similar nucleotide sequences and their fragments. Likewise, the amino acid sequence represented by each SEQ ID NO: includes the above-described various fragments thereof, similar nucleotide sequences and their fragments, and modified derivatives thereof. In addition, unless otherwise stated, BSSP4, hBSSP4, and mBSSP4 include proteins having the above-described respective amino acid sequences.

### BRIEF DESCRIPTION OF THE DRAWINGS

Fig. 1 illustrates the results of northern blotting using human multiple tissue blot membrane.

Fig. 2 illustrates the results of northern blotting using human multiple tissue blot II membrane.

Fig. 3 illustrates the results of northern blotting using human multiple tissue blot II membrane.

Fig. 4 illustrates the results of northern blotting using mRNA prepared in Example 2 hereinafter.

Fig. 5 illustrates the results of northern blotting using mRNA prepared in Example 2 hereinafter.

Fig. 6 illustrates the plasmid constructed by the method of Example 4 hereinafter.

Fig. 7 illustrates the construction of the plasmid by the method of Example 4 hereinafter.

### DETAILED DESCRIPTION OF THE INVENTION

The nucleotide sequences encoding hBSSP4 or mBSSP4 of the present invention can be obtained by preparing mRNAs from cells expressing the protein and converting it into double stranded DNAs according to a conventional manner. For preparing mRNA, guanidine isothiocyanate-calcium chloride method (Chirwin, et al., Biochemistry, 18, 5294, 1979) or,the like can be used. For preparing poly (A) + RNA from total RNAs, there can be used affinity chromatography using a carrier, for example, Sepharose, latex particles, etc., to which oligo (dT) is attached, and the like. The above-obtained RNA can be used as a template and treated with reverse transcriptase by using, as a primer, oligo (dT) which is complementary to the poly (A) strand at the 3'-terminus, or a random primer, or a synthesized oligonucleotide corresponding to a part of the amino acid sequence of hBSSP4 or mBSSP4 to obtain a hybrid mRNA strand comprising DNA complementary to the mRNA or cDNA. The double stranded DNA can be obtained by treating the above-obtained hybrid mRNA strand with *E. coli* RNase, *E. coli* DNA polymerase and *E. coli* DNA ligase to convert into a DNA strand.

It is also possible to carry out cloning by RT-PCR method using primers synthesized on the basis of the nucleotide sequence of hBSSP4 or mBSSP4 gene and using hBSSP4 or mBSSP4 expressing cell poly (A) + RNA as a template. Alternatively, the desired cDNA can be obtained without using PCR by preparing or synthesizing a probe on the basis of the nucleotide sequence of hBSSP4 or mBSSP4 gene and screening a cDNA library directly. Among genes obtained by these methods, the gene of the present invention can be selected by confirming a nucleotide sequence thereof. The gene of the present invention can also be prepared according to a conventional method using chemical syntheses of nucleic acids, for example, phosphoamidite method (Mattencci, M. D. et al., J. Am. Chem. Soc., 130, 3185, 1981) and the like.

By using the thus-obtained hBSSP4 or mBSSP4 gene, their expression in various tissues can be examined.

In case of northern blotting analysis, the expression of hBSSP4 is observed in cerebellum and prostate, and the expression of mBSSP4 is observed in prostate and skeletal muscle. In case of RT-PCR analysis, the expression of hBSSP4 is observed in brain, placenta and prostate of human fetuses and adults and the expression of mBSSP4 is observed in brain and placenta of 12-day-old mice. Then, the novel proteases of the present invention are presumed to play various roles in brain, prostate, placenta and skeletal muscle. For example, in brain, there is a possibility that they can be used for treatment and diagnosis of brain diseases such as Alzheimer's disease (AD), epilepsy, brain tumor and the like. Further, in other tissues, there is a possibility that they can be used for treatment and diagnosis of various diseases such as cancer, inflammation, sterility, prostate hypertrophy and the like. Further, it is presumed they may have a certain influence on blood coagulation, fibrinolysis and complement systems.

The human novel serine protease (hBSSP4) is composes of 9 proteins due to alternative splicing of mRNA.

The protein having the amino acid sequence represented by SEQ ID NO: 2 is a human type protein (hBSSP4) and the mature type having serine protease activity is the polypeptide represented by the 1st to 268th amino acids. As consensus sequences of serine proteases, it has Ala-Ala-His-Cys represented by the 39th to 42nd amino acids and Asp-Ser-Gly-Gly-Pro represented by the 192nd to 196th amino acids and one or more of Asp's are present between the concensus sequences. A nucleotide sequence encoding this protein is shown in SEQ ID NO: 1.

The protein having the amino acid sequence represented by SEQ ID NO: 4 is a human type protein (hBSSP4) and the mature type having serine protease activity is the polypeptide represented by the 1st to 270th amino acids. As consensus sequences of serine proteases, it has Ala-Ala-His-Cys represented by the 39th to 42nd amino acids and Asp-Ser-Gly-Gly-Pro represented by the 192nd to 196th amino acids and one or more of Asp's are present between the concensus sequences. A nucleotide sequence encoding this protein is shown in SEQ ID NO: 3. This sequence corresponds to SEQ ID NO: 1 from which the 943rd to 1217th bases have been removed, and the amino acid sequence represend by SEQ ID NO: 4 corresponds to the amino acid sequence represented by SEQ ID NO: 2 in which the 265th amino acid and the subsequent amino acids are different.

The protein having the amino acid sequence represented by SEQ ID NO: 6 is a human type protein (hBSSP4) and the mature type having serine protease activity is the polypeptide represented by the 1st to 257th amino acids. As consensus sequences of serine proteases, it has Ala-Ala-His-Cys represented by the 39th to 42nd amino acids and Asp-Ser-Gly-Gly-Pro represented by the 192nd to 196th amino acids and one or more of Asp's are present between the concensus sequences. A nucleotide sequence encoding this protein is shown in SEQ ID NO: 5. This sequence correponds to SEQ ID NO: 1 from which the 895th to 11208th bases have been removed, and the amino acid sequence represented by SEQ ID NO: 6 correspond to the amino acid sequence represente by SEQ ID NO: 2 in which the 249th amino acids and the subsequnent amino acids are different. Further, the nucleotide sequence corresponds to the sequence wherein the 969th to 1036th bases of SEQ ID NO: 5 are added to the downstream of the 1282 base of SEQ ID NO: 1.

The protein having the amino acid sequence represented by SEQ ID NO: 8 is a human type protein (hBSSP4). However, it does not have a consensus sequence of serine proteases. Since its expression by mRNA has been confirmed, this sequence is consiered to have a certain role. The nucleotide sequence corresponds to the nucleotide sequence of SEQ ID NO: 1 from which the 233rd to 282nd bases have been removed.

The protein having the amino acid sequence represented by SEQ ID NO: 10 is a human type protein (hBSSP4). As a consensus sequence of serine proteases, this does not have Ala-Ala-His-Cys represented, but has Asp-Ser-Gly-Gly-Pro represented by the 82nd to 86h amino acids. A nucleotide sequence encoding this protein is shown in SEQ ID NO: 9. This sequence corresponds to the nucleotide sequence of SEQ ID NO: 1 from which the 233rd to 562nd bases have been removed.

The protein having the amino acid sequence represented by SEQ ID NO: 12 is a human type protein (hBSSP4). As a consensus sequence of serine proteases, it has Ala-Ala-His-Cys represented by the 39th to 42nd amino acids but does not have Asp-Ser-Gly-Gly-Pro. A nucleotide sequence encoding this protein is shown in SEQ ID NO: 11. This nucleotide sequence corresponds to the nucleotide sequence represented by SEQ ID NO: 1 from which the 364th to 562nd amino acids have been removed.

The protein having the amino acid sequence represented by SEQ ID NO: 14 is a human type protein (hBSSP4). As a consensus sequence of serine proteases, it has Ala-Ala-His-Cys represented by the 39th to 42nd amino acids but do not have Asp-Ser-Gly-Gly-Pro. A nucleotide sequence encoding this protein is shown in SEQ ID NO: 13. This nucleotide sequence corresponds to the nucleotide sequence represented by SEQ ID NO: 1 from which the 588th to 1145th bases have been removed. There is a possibility that the nucleotide sequence represented by the 652nd and the subsequent bases of SEQ ID NO: 13 would be "ccc ggg ccc cag cgc ttt tgt gta tat aaa tgt taatgatttt tataggtatt tgtaaccctg cccacatatc" and the amino acid sequence represented by the 168th and the subsequent amino acids of SEQ ID NO: 14 would be "Pro Gly Pro Gln Arg Phe Cys Val, Tyr Lys Cys".

The protein having the amino acid sequence represented by SEQ ID NO: 16 is a human type protein (hBSSP4). As a consensus sequence of serine proteases, it has Ala-Ala-His-Cys represented by the 39th to 42nd amino acids but does not have Asp-Ser-Gly-Gly-Pro. A nucleotide sequence encoding this protein is shown in SEQ ID NO: 15. This sequence corresponds to SEQ ID NO: 1 from which the 285th to 562nd bases have been removed.

The protein having the amino acid sequence represented by SEQ ID NO: 18 is a human type protein (hBSSP4). As a consensus sequence of serine proteases, it has Ala-Ala-His-Cys represented by the 39th to 42nd amino acids but does not have Asp-Ser-Gly-Gly-Pro. A nucleotide sequence encoding this protein is shown in SEQ ID NO: 17. This sequence corresponds to the sequence wherein the 721st to 948th bases of SEQ ID NO: 17 is added to the downstream of the 720th base of SEQ ID NO: 1, and corresponds SEQ ID NO: 1 from which the 720th and the subsequent bases have been removed.

The protein having the amino acid sequence rèpresented by SEQ ID NO: 20 is a mouse type protein (mBSSP4) and the mature type having serine protease activity is the polypeptide represented by the 1st to 253 amino acids. As consensus sequences of serine proteases, it has Ala-Ala-His-Cys represented by the 39th to 42nd amino acids and Asp-Ser-Gly-Gly-Pro represented by the 192nd to 196th amino acids and one or more of Asp's are present between the concensus sequences. A nucleotide sequence encoding this protein is shown in SEQ ID NO: 19.

The term "pro part" used herein means a part of a pro-form, i.e., the pro-form from which the corresponding active type protein part is removed. The term "pre part" used herein means a part of a prepro-form, i.e., the prepro-form from which the corresponding pro-form is removed. The term "prepro part" used herein means a part of a prepro-form, i.e., the prepro-form from which the corresponding active type protein part is removed.

The amino acid sequence of mature hBSSP4 (the 1st to 268th amino acids) represented by SEQ ID NO: 2 is hBSSP4 mature or active type protein composed of 268 amino acids, and the nucleotide sequence encoding the amino acid sequence is composed of 804 bases. The present inventors have shown that the serine protease activity is maintained even when one to several amino acids of the N-terminus of the mature type protein of the present invention is deleted or added, while the preferred sequence is this amino acid sequence. The sequence of the -49th to -1st amino acids is the prepro or pro part and the amino acid sequence of the -15th to -1st amino acids is the pro part and is considered to be a precursor of hBSSP4 protein.

The amino acid sequence of mature hBSSP4 (the 1st to 270th amino acids) represented by SEQ ID NO: 4 is hBSSP4 mature or active type protein composed of 270 amino acids, and the nucleotide sequence encoding the amino acid sequence is composed of 810 bases. The present inventors have shown that the serine protease activity is maintained even when one to several amino acids of the N-terminus of the mature type protein of the present invention is deleted or added, while the preferred sequence is this amino acid sequence. The sequence of the -49th to -1st amino acids is the prepro or pro part and the amino acid sequence of the -15th to -1st amino acids is the pro part and is considered to be a precursor of hBSSP4 protein.

The amino acid sequence of mature hBSSP4 (the 1st to 257th amino acids) represented by SEQ ID NO: 6 is hBSSP4 mature or active type protein composed of 257 amino acids, and the nucleotide sequence encoding the amino acid sequence is composed of 771 bases. The present inventors have shown that the serine protease activity is maintained even when one to several amino acids of the N-terminus of the mature type protein of the present invention is deleted or added, while the preferred sequence is this amino acid sequence. The sequence of the -49th to -1st amino acids is the prepro or pro part and the amino acid sequence of the -15th to -1st amino acids is the pro part and is considered to be a precursor of hBSSP4 protein.

The amino acid sequence of hBSSP4 (the 1st to 97th amino acids) represented by SEQ ID NO: 8 is a protein composed of 97 amino acids, and the nucleotide sequence encoding the amino acid sequence is composed of 291 bases. The sequence of the -49th to -1st amino acids is the prepro or pro part and the amino acid sequence of the -15th to -1st amino acids is the pro part and is considered to be a precursor of hBSSP4 protein.

The amino acid sequence of hBSSP4 (the 1st to 158th amino acids) represented by SEQ ID NO: 10 is a protein composed of 158 amino acids, and the nucleotide sequence encoding the amino acid sequence is composed of 474 bases. The sequence of the -49th to -1st amino acids is the prepro or pro part and the amino acid sequence of the -15th to -1st amino acids is the pro part and is considered to be a precursor of hBSSP4 protein.

The amino acid sequence of hBSSP4 (the 1st to 82nd amino acids) represented by SEQ ID NO: 12 is a protein composed of 82 amino acids, and the nucleotide sequence encoding the amino acid sequence is composed of 246 bases. The sequence of the -49th to -1st amino acids is the prepro or pro part and the amino acid sequence of the -15th to -1st amino acids is the pro part and is considered to be a precursor of hBSSP4 protein.

The amino acid sequence of hBSSP4 (the 1st to 185th amino acids) represented by SEQ ID NO: 14 is a protein composed of 185 amino acids, and the nucleotide sequence encoding the amino acid sequence is composed of 555 bases. The sequence of the -49th to -1st amino acids is the prepro or pro part and the amino acid sequence of the -15th to -1st amino acids is the pro part and is considered to be a precursor of hBSSP4 protein.

The amino acid sequence of hBSSP4 (the 1st to 80th amino acids) represented by SEQ ID NO: 16 is a protein composed of 80 amino acids, and the nucleotide sequence encoding the amino acid sequence is composed of 240 bases. The sequence of the -49th to -1st amino acids is the prepro or pro part and the amino acid sequence of the -15th to -1st amino acids is the pro part and is considered to be a precursor of hBSSP4 protein.

The amino acid sequence of hBSSP4 (the 1st to 253th amino acids) represented by SEQ ID NO: 18 is a protein composed of 253 amino acids, and the nucleotide sequence encoding the amino acid sequence is composed of 759 bases. The sequence of the -49th to -1st amino acids is the prepro or pro part and the amino acid sequence of the -15th to -1st amino acids is the pro part and is considered to be a precursor of hBSSP4 protein.

The amino acid sequence of mature mBSSP4 (the 1st to 259th amino acids) represented by SEQ ID NO: 20 is hBSSP4 mature or active type protein composed of 259 amino acids, and the nucleotide sequence encoding the amino acid sequence is composed of 777 bases. The present inventors have shown that the serine protease activity is maintained even when one to several amino acids of the N-terminus of the mature type protein of the present invention is deleted or added, while the preferred sequence is this amino acid sequence. The sequence of the -49th to -1st amino acids is the prepro or pro part and the amino acid sequence of the -15th to -1st amino acids is the pro part and is considered to be a precursor of mBSSP4 protein.

In general, many genes of eucaryote exhibit polymorphism and, sometimes, one or more amino acids are substituted by this phenomenon. Further, even in such case, sometimes, a protein maintains its activity. Then, the present invention includes a gene encoding a protein obtained by modifying a gene encoding any one of the amino acid sequences represented by SEQ ID NOS: 2, 4, 6, 8, 10, 12, 14, 16, 18 and 20, artificially, in so far as the protein has the characteristic function of the gene of the present invention. Further, the present invention includes a protein which is a modification of any one of amino acid sequences represented by SEQ ID NOS: 2, 4, 6, 8, 10, 12, 14, 16, 18 and 20 in so far as the protein has the characteristics of the present invention. Modification is understood to include substitution, deletion, addition and/or insertion. In particular, the present inventors have shown that, even when several amino acids are added to or deleted from the N-terminus amino acid of the hBSSP4 or mBSSP4 mature protein represented by SEQ ID NO: 2, 4, 6 or 20, the resultant sequence maintains its activity.

That is, the present invention includes a protein comprising any one of amino acid sequences described in SEQ ID NOS: 2, 4, 6, 8, 10, 12, 14, 16, 18 and 20; an amino acid sequence encoded by any one of nucleotide sequences represented by SEQ ID NOS: 1, 3, 5, 7, 9, 11, 13, 15, 17 and 19; or one of these amino acid sequences wherein one to several amino acids have been substituted, deleted, added and/or inserted, and being belonging to serine protease family.

Each codon for the desired amino acid itself has been known and it can be selected freely. For example, codons can be determined according to a conventional manner by taking into consideration of frequency of use of codons in a host to be utilized (Grantham, R. et al., Nucleic Acids Res., 9, r43, 1989). Therefore, the present invention also includes a nucleotide sequence appropriately modified by taking into consideration of degeneracy of a codon. Further, these nucleotide sequences can be modified by a site directed mutagenesis using a primer composed of a synthetic oligonucleotide encoding the desired modification (Mark, D. F. et al., Proc. Natl. Acad. Sci. USA., 81, 5662, 1984), or the like.

Furthermore, the DNA of the present invention includes DNA which is hybridizable to any one of nucleotide sequences described in SEQ ID NOS: 1, 3, 5, 7, 9, 11, 13, 15, 17 and 19 or nucleotide sequences complementary to these nucleotide sequences in so far as the protein encoded by the nucleotide sequence has the same properties as those of hBSSP4 or mBSSP4 of the present invention. It is considered that many of sequences which are hybridizable to a given sequence under stringent conditions have a similar activity to that of a protein encoded by the given sequence. The stringent conditions according to the present invention includes, for example, incubation in a solution containing 5 x SSC, 5% Denhardt's solution (0.1% BSA, 0.1% Ficol 1400, 0.1% PVP), 0.5% SDS and 20 µg/ml denatured salmon sperm DNA at 37°C overnight, followed by washing with 2 x SSC containing 0.1% SDS at room temperature. Instead of SSC, SSPE can be appropriately used.

Probes for detecting a hBSSP4 or mBSSP4 gene can be designed based on any one of nucleotide sequences described in SEQ ID NOS: 1, 3, 5, 7, 9, 11, 13, 15, 17 and 19. Or, primers can be designed for amplifying DNA or RNA containing the nucleotide sequence. To design probes or primers is carried out routinely by a person skilled in the art. An oligonucleotide having a designed nucleotide sequence can be synthesized chemically. And, when a suitable label is added to the oligonucleotide, the resultant oligonucleotide can be utilized in various hybridization assay. Or, it can be utilized in nucleic acid synthesis reactions such as PCR. An oligonucleotide to be utilized as a primer has, preferably, at least 10 bases, more preferably 15 to 50 bases in length. An oligonucleotide to be utilized as a probe has, preferably, 100 bases to full length.

Moreover, it is possible to obtain a promoter region and an enhancer region of a hBSSP4 or mBSSP4 gene present in the genome based on the cDNA nucleotide sequence of hBSSP4 or mBSSP4 provided by the present invention. Specifically, these control regions can be obtained according to the same manner as described in JP 6-181767 A; J. Immunol., 155, 2477, 1995; Proc. Natl. Acad. Sci., USA, 92, 3561, 1995 and the like. The promoter region used herein means a DNA region which is present upstream from a transcription initiation site and controls expression of a gene. The enhancer region used herein means a DNA region which is present in an intron, a 5'-non-translated region or a 3'-non-translated region and enhances expression of a gene.

The present invention also relates to a vector comprising the nucleotide sequence represented by SEQ ID NO: 1, 3, 5, 7, 9, 11, 13, 15, 17 or 19, or a nucleotide sequence encoding the amino acid sequence represented by SEQ ID NO:' 2, 4, 6, 8, 10, 12, 14, 16, 18 ir 20, or a nucleotide sequence similar to these sequences. A nucleotide sequence similar to a give nucleotide sequence used herein means a nucleotide sequence which is hybridizable to the given nucleotide sequence or its complementary nucleotide sequence under the above-described stringent conditions and encodes a protein having the same properties as those of the protein encoded by the nucleotide sequence.

The vector is not specifically limited in so far as it can express the protein of the present invention. Examples thereof include pBAD/His, pRSETA, pcDNA2.1, pTrcHis2A, pYES2, pBlueBac4.5, pcDNA3.1 and pSecTag2 manufacture by Invitrogen, pET and pBAC manufactured by Novagen, pGEM manufactured by Promega, pBluescriptII manufactured by Stratagene, pGEX and pUC18/19 manufactured by Pharmacia, PfastBAC1 manufactured by GIBCO and the like. Preferably, a protein expression vector (described in the specification of a patent application entitled "Protein expression vector and its use" and filed by the same applicant on the same day) is used. This expression vector is constructed by using pCRII-TOPO vector described in the Examples hereinafter, or a commercially available expression vector, for example pSecTag2A vector or pSecTag2B vector (Invitrogen) and integrating a secretory signal nucleotide sequence suitable for expression of the protein of the present invention, in the 3' downstream side thereof, a Tag nucleotide sequence, a cleavable nucleotide sequence and a cloning site, into which a nucleotide sequence encoding a target protein can be inserted, in this order. More specifically, it is preferred to use trypsin signal as the secretory signal, a nucleotide sequence encoding polyhistidine as the Tag nucleotide sequence, and a nucleotide sequence encoding an amino acid sequence which is susceptible to enzyme-specific cleavage, i.e., a nucleotide sequence encoding the amino acid sequence of Asp-Asp-Asp-Asp-Lys (said amino acid sequence is recognized by enterokinase, and the recombinant fusion protein is cleaved at the C-terminus part thereof) as the cleavable nucleotide sequence.

Furthermore, the present invention provides transformed cells having the nucleotide sequence of the present invention in an expressible state by means of the above vector. Preferably, host cells to be used for the transformed cells of the present invention are animal cells and insect cells. However, host cells include any cells (including those of microorganisms) which can express a nucleotide sequence encoding the desired protein in the expression vector of the present invention and can secrete extracellularly.

The animal cells and insect cells used herein include cells derived from human being and cells derived from fly or silk worm. For example, there are CHO cell, COS cell, BHK cell, Vero cell, myeloma cell, HEK293 cell, HeLa cell, Jurkat cell, mouse L cell, mouse C127 cell, mouse FM3A cell, mouse fibroblast, osteoblast, cartilage cell, S2, Sf9, Sf21, High Five™ (registered trade mark) cell and the like. The microorganisms used herein include *E. coli* and yeast.

The protein of the present invention as such can be expressed as a recombinant fused protein so as to facilitate isolation, purification and recognition. The recombinant fused protein used herein means a protein expressed as an adduct wherein a suitable peptide chain are added to the N-terminus and/or C-terminus of the desired protein expressed by a nucleotide sequence encoding the desired protein. The recombinant protein used herein means that obtained by integrating a nucleotide sequence encoding the desired protein in the expression vector of the present invention and cut off an amino acid sequence which derived from nucleic acids other than those encoding the desired protein from the expressed recombinant fused protein, and is substantially the same as the protein of the present invention.

Introduction of the above vector into host cells can be carried out by, for example, transfection according to' lipopolyamine method, DEAE-dextran method, Hanahan method, lipofectin method or calcium phosphate method, microinjection, eletroporation and the like.

As described above, the present invention also relates to a process for producing hBSSP4 of mBSSP4 comprising culturing cells transformed with the above nucleotide sequence of the present invention and collecting the produced hBSSP4 of mBSSP4. The culture of cells and separation and purification of the protein can be carried out by a per se known method.

The present invention also relates to an inhibitor of the novel serine protease of the present invention. Screening of the inhibitor can be carried out according to a per se known method such as comparing the enzyme activity upon bringing into contact with a candidate compound with that without contact with the candidate compound, or the like

The present invention relates to a non-human transgenic animal whose expression level of hBSSP4 or mBSSP4 gene has been altered. The hBSSP4 or mBSSP4 gene used herein includes cDNA, genomic DNA or synthetic DNA encoding hBSSP4 or mBSSP4. In addition, expression of a gene includes any steps of transcription and translation. The non-human transgenic animal of the present invention is useful for studies of functions or expression control of hBSSP4 or mBSSP4, elucidation of mechanisms of diseases in which hBSSP4 or mBSSP4 is presumed to be involved, and development of disease model animals for screening and safety test of medicine.

In the present invention, expression of a gene can be modified artificially by mutagenizing at a part of several important sites which control normal gene expression (enhancer, promoter, intron, etc.) such as deletion, substitution, addition and/or insertion to increase or decrease an expression level of the gene in comparison with its inherent expression level. This mutagenesis can be carried out according to a known method to obtain the transgenic animal.

In a narrow sense, the transgenic animal means an animal wherein a foreign gene is artificially introduced into reproductive cells by gene recombinant techniques. In a broad sense, the transgenic animal includes an antisense transgenic animal the function of whose specific gene is inhibited by using antisense RNA, an animal whose specific gene is knocked out by using embryonic stem cells (ES cells), and an animal into which point mutation DNA is introduced, and the transgenic animal means an animal into which a foreign gene is stably introduced into a chromosome at an initial stage of ontogeny and the genetic character can be transmitted to the progeny.

The transgenic animal used herein should be understood in a broad sense and includes any vertebrates other than a human being. The transgenic animal of the present invention is useful for studies of functions or expression control of hBSSP4 or mBSSP4, elucidation of mechanisms of diseases associated with cells expressing in a human being, and development of disease model animals for screening and safety test of medicine.

As a technique for creating the transgenic animal, a gene is introduced into a nucleus in a pronucleus stage of egg cells with a micropipette directly under a phase-contrast microscope (microinjection, U.S. Patent 4,873,191). Further, there are a method using embryonic stem cell (ES cell), and the like. In addition, there are newly developed methods such as a method wherein a gene is introduced into a retroviral vector or adenoviral vector to infect egg cells, a sperm vector method wherein a gene is introduced into egg cells through sperms, and the like.

A sperm vector method is a gene recombinant technique wherein a foreign gene is incorporated into sperm cells by adhesion, electroporation, etc., followed by fertilization of egg cells to introduce the foreign gene into the egg cells (M. Lavitranoet et al., Cell, 57, 717, 1989). Alternatively, an in vivo site specific gene recombinant technique such as that using cre/loxP recombinase system of bacteriophage P1, FLP recombinase system of *Saccharomyces cerevisiae,* etc. can be used. Furthermore, introduction of a transgene of the desired protein into a non-human animal using a retroviral vector has been reported.

For example, a method for creating a transgenic animal by microinjection can be carried out as follows.

First, a transgene primarily composed of a promoter responsible for expression control, a gene encoding a specific protein and a poly A signal is required. It is necessary to confirm expression modes and amounts between respective systems because an expression mode and amount of a specific molecule is influenced by a promoter activity, and transgenic animals differ from each other according to a particular system due to the difference in a copy number of an introduced transgene and a introduction site on a chromosome. An intron sequence which is spliced may be previously introduced before the poly A signal because it has been found that an expression amount varies due to a non-translation region and splicing. Purity of a gene to be used for introduction into fertilized egg cells should be as high as possible. This is of importance. Animals to be used include a mouse for collecting fertilized eggs (5 to 6 week old), a male mouse for mating, a false pregnancy female mouse, a seminiferous tubule-ligated mouse, and the like.

For obtaining fertilized egg cells efficiently, ovulation may be induced with gonadotropin or the like. Fertilized egg cells are recovered and a gene in an injection pipette is injected into male pronucleus of the egg cells by microinjection. For returning the injected egg cells to a fallopian tube, an animal (false pregnancy female mouse, etc.) is provided and about 10 to 15 eggs/mouse are transplanted. Then, genomic DNA is extracted from the end part of the tail to confirm whether the transgene is introduced into newborn mouse or not. This confirmation can be carried out by detection of the transgene with southern blot technique or PCR technique, or by positive cloning wherein a marker gene, which is activated only when homologous recombination is caused, has been introduced. Further, transcribed products derived from the transgene are detected by northern blot technique or RT-PCR technique to confirm expression of the transgene. Or, western blotting can be carried out with a specific antibody to a protein.

The knockout mouse of the present invention is treated so that the function of mBSSP4 gene is lost. A knockout mouse means a transgenic mouse any of whose gene is destroyed by homologous recombination technique so that its function is deficient. A knockout mouse can be created by carrying out homologous recombination with ES cells and selecting embryonic stem cells wherein either of allele genes are modified or destroyed. For example, embryonic stem cells whose genes are manipulated at blastocyte or morula stage of fertilized eggs are injected to obtain a chimera mouse wherein cells derived from the embryonic stem cells are mixed with those derived from the embryo. The chimera mouse (chimera means a single individual formed by somatic cells based on two or more fertilized eggs) can be mated with a normal mouse to create a heterozygote mouse wherein all of either of the allele genes have been modified or destroyed. Further, a homozygote mouse can be created by mating heterozygote mice.

Homologous recombination means recombination between two genes whose nucleotide sequences are the same or very similar to each other in terms of gene recombination mechanism. PCR can be employed to select homologous recombinant cells. A PCR reaction can be carried out by using a part of a gene to be inserted and a part of a region where the insertion is expected as primers to find out occurrence of homologous recombination in cells which give an amplification product. Further, for causing homologous recombination in a gene expressed in embryonic stem cells, homologous recombinant cells can readily be selected by using a known method or its modification. For example, cells can be selected by joining a neomycin resistant gene to a gene to be introduced to impart neomycin resistance to cells after introduction.

The present invention also provide an antibody recognizing hBSSP4 or mBSSP4 or a fragment thereof. The antibody of the present invention includes an antibody against a protein having the amino acid sequence described in any of SEQ ID NOS: 2, 4, 6, 18 and 20 or its fragment. An antibody against hBSSP4 or mBSSP4 or a fragment thereof (e.g., polyclonal antibody, monoclonal antibody, peptide antibody) or an antiserum can be produced by using hBSSP4 or mBSSP4 or a fragment thereof, etc. as an antigen according to a per se known process for producing an antibody or an antiserum.

The hBSSP4 or mBSSP4 or a fragment thereof is administered to a site of a warm-blooded animal where an antibody can be produced by administration thereof as such or together with a diluent or carrier. For enhancing the antibody production, upon administration, Freund's complete adjuvant or Freund's incomplete adjuvant may be administrated. Normally, the administration is carried out once every 1 to 6 weeks, 2 to 10 times in all. Examples of the warm-blooded to be used include monkey, rabbit, dog, guinea pig, mouse, rat, sheep, goat, chicken and the like with mouse and rat being preferred. As rats, for example, Wistar and SD rats are preferred. As mice, for example, BALB/c, C57BL/6 and ICR mice are preferred.

For producing monoclonal antibody producer cells, individuals whose antibody titer have been recognized are selected from warm-blooded animals, e.g., a mouse immunized with an antigen. Two to 5 days after the last immunization, the spleen or lymph node of the immunized animal is collected and antibody producer cells contained therein are subjected to cell fusion with myeloma cells to prepare a monoclonal antibody producer hybridoma. The antibody titer in an antiserum can be determined by, for example, reacting the antiserum with a labeled hBSSP4 or mBSSP4 as described hereinafter, followed by measurement of the activity bound to the antibody. The cell fusion can be carried out according to a known method, for example, that described by Koehler and Milstein (Nature, 256, 495, 1975) or its modifications (J. Immunol. Method, 39, 285, 1980; Eur. J. biochem, 118, 437, 1981; Nature, 285, 446, 1980). As a fusion promoting agent, there are polyethylene glycol (PEG), Sendai virus and the like. Preferably, PEG is used. Further, for improving fusion efficiency, lectin, poly-L-lysine or DMSO can be appropriately added.

Examples of myeloma cells include X-63Ag8, NS-1, P3U1, SP2/0, AP-1 and the like with SP2/0 being preferred. The preferred ratio of the number of the antibody producer cells (spleen cells) : the number of spleen cells are 1 : 20 to 20 : 1. PEG (preferably PEG 1000 to PEG 6000) is added at a concentration of about 10 to 80% and the mixture is incubated at 20 to 40°C, preferably 30 to 37°C for 1 to 10 minutes to carry out the cell fusion efficiently. Screening of anti-hBSSP4 or mBSSP4 antibody producer hybridomas can be carried out by various methods. For example, a supernatant of a hybridoma culture is added to a solid phase to which hBSSP4 or mBSSP4 antigen is adsorbed directly or together with a carrier (e.g., microplate), followed by addition of an anti-immunoglobulin antibody (in case that the cells used in cell fusion is those of a mouse, anti-mouse immunoglobulin antibody is used) or protein A to detect the anti-hBSSP4 or mBSSP4 monoclonal antibody attached to the solid phase. Or, a supernatant of a hybridoma culture is added to a solid phase to which an anti-immunoglobulin antibody or protein A is adsorbed, followed by addition of hBSSP4 or mBSSP4 labeled with a radioactive substance, an enzyme, etc., to detect the anti-hBSSP4 or mBSSP4 monoclonal antibody attached to the solid phase.

Selection and cloning of the anti-hBSSP or mBSSP monoclonal antibody can be carried out according to a per se known method or its modification. Normally, a HAT (hypoxanthine, aminopterin, thymidine)-added medium for culturing animal cells is used. Any culture medium can be used for selection, cloning and growing up in so far as the hybridoma can grow. For example, there can be used RPMI culture medium containing 1 to 20%, preferably 10 to 20% fetal bovine serum, or a serum-free medium for culturing hybridomas. Preferably, the culture is carried out at a temperature of about 37°C. Normally, the culture time is 5 days to 3 weeks, preferably 1 weeks to 2 weeks. Normally, the culture is carried out under 5% CO₂. The antibody titer of a supernatant of a hybridoma culture can be measured according to the same manner as that of the above-described measurement of anti-BSSP4 antibody titer in an antiserum. That is, examples of the measurement to be used include radioimmunoassay (RIA), enzyme-linked immunosorbent assay (ELISA), FIA (fluorescence immunoassay), plaque assay, agglutination reaction method, and the like. Among them, ELISA as shown blew is preferred.

### Screening by ELISA

A protein prepared according to the same operation as that for an immunogen is immobilized on the surface of each well of an ELISA plate. Next, BSA, MSA, OVA, KLH, gelatin, skimmed milk, or the like is immobilized on each well to prevent non-specific adsorption. A supernatant of a hybridoma culture is added to each well and is allowed to stand for a given time so that an immunological reaction proceeds. Each well is washed with a washing solution such as PBS or the like. Preferably, a surfactant is added to this washing solution. An enzyme labeled secondary antibody is added and allowed to stand for a given time. As the enzyme to be used for the label, there can be used β-galactosidase, alkaline phosphatase, peroxidase and the like. After washing each well with the same washing solution, a substrate solution of the labeled enzyme used is added so that an enzymatic reaction proceeds. When the desired antibody is present in the supernatant of a hybridoma culture, the enzymatic reaction proceeds and the color of the substrate solution is changed.

Normally, cloning is carried out by a per se known method such as semi-solid agar method, limiting dilution method and the like. Specifically, after confirming a well in which the desired antibody is produced by the above-described method, cloning is carried out to obtain a single clone. For cloning, it is preferred to employ limiting dilution method wherein hybridoma cells are diluted so that one colony is formed per one well of a culture plate. For cloning by limiting dilution method, feeder cells can be used, or a cell growth factor such as interleukin 6, etc. can be added to improve colony forming capability. In addition, cloning can be carried out by using FACS and single cell manipulation method. The cloned hybridoma is preferably cultured in a serum-free culture medium and an optimal amount of an antibody is added to its supernatant. The single hybridoma thus obtained can be cultured in a large about by using a flask or a cell culture device, or cultured in the abdominal cavity of an animal (J. Immunol. Meth., 53, 313, 1982) to obtain a monoclonal antibody. When culturing in a flask, there can be used a cell culture medium (e.g., IMDM, DMEM, RPMI1640, etc.) containing 0 to 20% of FCS. When culturing in the abdominal cavity of an animal, the animal to be used is preferably the same species or the same line as that from which the myeloma cells used in the cell fusion are derived, a thymus deficient nude mouse or the like, and the hybridoma is transplanted after administration of a mineral oil such as pristane, etc. After 1 to 2 weeks, myeloma cells are proliferated in the abdominal cavity to obtain ascites containing a monoclonal antibody.

The monoclonal antibody of the present invention which does not cross-react with other proteins can be obtained by selecting a monoclonal antibody which recognizes an epitope specific to hBSSP4 or mBSSP4. In general, an epitope presented by an amino acid sequence composed of at least 3, preferably 7 to 20 successive amino acid residues in an amino acid sequence which constitutes a particular protein is said to be an inherent epitope of the protein. Then, a monoclonal antibody recognizing an epitope constituted by a peptide having an amino acid sequence composed of at least 3 successive amino acid residue selected from the amino acid residues disclosed in any of SEQ ID NOS: 2 and 4 can be said to be the monoclonal antibody specific for hBSSP4 or mBSSP4 of the present invention. An epitope common to BSSP4 family can be selected by selecting an amino acid sequence conservative among the amino acid sequences described in SEQ ID NOS: 2 and 4. Or, in case of a region containing an amino acid sequence specific for each sequence, a monoclonal antibody which can differentiate respective proteins can be selected.

Separation and purification of the anti-hBSSP4 or mBSSP4 monoclonal antibody, like a conventional polyclonal antibody, can be carried out according to the same manner as those of immunoglobulins. As a known purification method, there can be used a technique, for example, salting out, alcohol precipitation, isoelectric precipitation, electrophoresis, ammonium sulfate precipitation,' absorption and desorption with an ion exchange material (e.g., DEAE), ultrafiltration, gel filtration, or specific purification by collecting only an antibody with an antibody-binding solid phase or an active adsorber such as protein A or protein G, etc., and dissociating the binding to obtain the antibody. For preventing formation of aggregates during purification or decrease in the antibody titer, for example, human serum albumin is added at a concentration of 0.05 to 2%. Alternatively, amino acids such as glycine, α-alanine, etc., in particular, basic amino acids such as lysine, arginine, histidine, etc., saccharides such as glucose, mannitol, etc., or salts such as sodium chloride, etc. can be added. In case of IgM antibody, since it is very liable to be aggregated, it may be treated with β-propionilactone and acetic anhydride.

The polyclonal antibody of the present invention can be produced according to a per se known method or its modification. For example, an immunogen (protein antigen) per se or a complex thereof with a carrier protein is prepared and, according to the same manner as that in the above monoclonal antibody production, a warm-blooded animal is immunized. A material containing an antibody against the protein of the present invention or its fragment is collected from the immunized animal and the antibody is separated and purified to obtain the desired antibody. As for a complex of an immunogen and a carrier protein for immunizing a warm-blooded animal, the kind of a carrier protein and the mixing ratio of a carrier and a hapten are not specifically limited in so far as an antibody against the hapten immunized by cross-linking with the carrier is efficiently produced. For example, there can be used about 0.1 to 20, preferably about 1 to 5 parts by weight of bovine serum albumin, bovine cycloglobulin, hemocyanin, etc. coupled with one part by weight of a hapten. For coupling a carrier and a hapten, various condensing agents can be used. Examples thereof include glutaraldehyde, carbodiimide or maleimide active ester, active ester agents having thiol group or dithiopyridyl group, and the like. The condensed product is administered as such or together with a carrier or diluent to a site of a warm-blooded animal where an antibody can be produced. For enhancing the antibody production, upon administration, Freund's complete adjuvant or Freund's incomplete adjuvant may be administrated. Normally, the administration is carried out once every 2 to 6 weeks, 3 to 10 times in all. The pblyclonal antibody can be collected from blood, ascites, or the like, preferably blood of the immunized animal. The polyclonal antibody titer in an antiserum can be measured according to the same manner as measurement of the above monoclonal antibody titer in the antiserum. Separation and purification of the polyclonal antibody, like the above monoclonal antibody, can be carried out according to the same manner as those of immunoglobulins.

The monoclonal antibody and polyclonal antibody against hBSSP4 or mBSSP4 or a fragment thereof can be utilized for diagnosis and treatment of diseases associated with cells expressing hBSSP4 or mBSSP4. By using these antibodies, hBSSP4 or mBSSP4 or a fragment thereof can be determined based on their immunological binding to hBSSP4 or mBSSP4 or a fragment thereof of the present invention. Specifically, examples of a method for determining hBSSP4 or mBSSP4 or a fragment thereof in a specimen by using these antibodies include a sandwich method wherein the antibody attached to an insoluble carrier and the labeled antibody are reacted with hBSSP4 or mBSSP4 or a fragment thereof to form a sandwich complex and the sandwich complex is detected, as well as a competitive method wherein labeled hBSSP4 or mBSSP4, and hBSSP4 or mBSSP4 or a fragment thereof in the specimen are competitively reacted with the antibody and hBSSP4 or mBSSP4 or a fragment thereof in the specimen is determined based on the amount of the labeled antigen reacted with the antibody.

As a sandwich method for determining hBSSP4 or mBSSP4 or a fragment thereof, there can be used two step method, one step method and the like. In two step method, first, the immobilized antibody is reacted with hBSSP4 or mBSSP4 or a fragment thereof and then unreacted materials are completely removed by washing, followed by addition of the labeled antibody to form immobilized antibody-hBSSP4 or mBSSP4-labeled antibody. In one step method, the immobilized antibody, labeled antibody and hBSSP4 or mBSSP4 or a fragment thereof are added at the same time.

Examples of an insoluble carrier used for the determination include synthetic resins such as polystyrene, polyethylene, polypropylene, polyvinyl chloride, polyester, polyacrylate, nylon, polyacetal, fluorine plastic, etc.; polysaccharides such as cellulose, agarose, etc.; glass; metal; and the like. An insoluble carrier may be shaped in various forms, for example, tray, sphere, fiber, rod plate, container, cell, test tube, and the like. The antibody adsorbed by a carrier is stored at a cold place in the presence of an appropriate preservative such as sodium azide or the like.

For immobilization of the antibody, a known chemical bonding method or a physical adsorption can be used. Examples of a chemical bonding method include 'a method using glutaraldehyde; maleimide method using N-succusinimidyl-4-(N-maleimidomethyl)cyclohexane-1-carboxylate, N-succusinimidyl-2-maleimide acetate or the like; carbodiimide method using 1-ethyl-3-(3-dimethylaminopropyl)carbodiimide hydrochloride; or the like. In addition, there are maleimidobenzoyl-N-hydroxysuccinimide ester method, N-succinimidyl-3-(2-pyridylthio)propionic acid method, bisdiazobenzidine method, and dipalmityllysine method. Or, it is possible to capture a complex formed beforehand by reacting a materiel to be tested with two antibodies, whose epitopes are different, with an immobilized a 3rd antibody against the antibody.

For labeling, it is preferred to use enzyme, fluorescent substance, luminous substance, radioactive substance, metal chelate, or the like. Examples of the enzyme include peroxidase, alkaline phosphatase, β-D-galactosidase, malate dehydrogenase, *Staphylococcus* nuclease, δ-5-steroidisomerase, α-glycerol phosphate dehydrogenase, triose phosphate isomerase, horseradish peroxidase, asparaginase, glucose oxidase, ribonuclease, urease, catalase, glucose-6-phosphate dehydrogenase, glucoamylase, acetylcholinesterase and the like. Examples of the fluorescent substance include fluorescein isothiocyanate, phycobiliprotein, rhodamine, phycoerythrin, phycocyanin, allophycocyanin, o-phthalaldehyde, and the like. Examples of the luminous substance include isoluminol, lucigenin, luminol, aromatic acridinium ester, imidazole, acrdinium salt and its modified ester, luciferin, luciferase, aequorin and the like. Examples of the radioactive substance include ¹²⁵I, ¹²⁷I, ¹³¹I, ¹⁴C, ³H, ³²P, ³⁵S and the like. The labeling material is not limited to them and any material which can be used for immunological determination can be used. Further, a low molecular weight hapten such as biotin, dinitrophenyl, pyridoxal or fluorescamine may be attached to the antibody. Preferably, horseradish peroxidase is used as a labeling enzyme. This enzyme can be reacted with various substrates and can readily be attached to the antibody by periodate method.

When an enzyme is used as a labeling material, a substrate and, if necessary, a coloring enzyme is used for measuring its activity. In case of using peroxidase as the enzyme, H₂O₂ is used as a substrate and, as a coloring agent, there can be used 2,2'-azino-di-[3-ethylbenzthiazoline sulfonic acid] ammonium salt (ABTS), 5'-aminosalicylic acid, o-phenylenediamine, 4-aminoantipyrine, 3,3',5,5'-tetramethylbenzidine and the like. In case of using alkaline phosphatase as the enzyme, o-nitorphenylphosphate, p-nitrophenylphosphoric acid, or the like can be used as a substrate. In case of using β-D-galactosidase as the enzyme, fluorescein-d-(β-D-galactopyranoside), 4-methylumbelliphenyl-β-D-galactopyranoside, or the like can be used as a substrate. The present invention also include a kit comprising the above monoclonal antibody, polyclonal antibody and reagents.

As a cross-linking agent, a known cross-linking agent such as N,N'-o-phenylenedimaleimide, 4-(N-maleimidomethyl)cyclohexanoate-N-succinimide ester, 6-maleimidohexanoate-N-succineimide ester, 4,4'-dithiopyridine or the like can be utilized. The reaction of these cross-linking agents with enzymes and antibodies can be carried out by a known method according to properties of a particular cross-linking agent. Further, as the antibody, a fragment thereof, for example, Fab', Fab, F(b'2) can be used as the case may be. A labeled enzyme can be obtained by the same treatment regardless of whether the antibody is polyclonal or monoclonal. When the above labeled enzyme obtained by using a cross-linking agent is purified by a known method such as affinity chromatography or the like, a immunoassay system having more higher sensitivity can be obtained. The enzyme labeled and purified antibody is stored at a dark cold place with addition of a stabilizer such as thimerosal, glycerin or after lyophilization.

An objective to be determined is not specifically limited in so far as it is a sample containing BSSP4 or a fragment thereof, or a sample containing a precursor of BSSP4 or a fragment thereof and includes body fluids such as plasma, serum, blood, serum, urine, tissue fluid, cerebrospinal fluid and the like.

The following Examples further illustrate the present invention in detail but are not construed to limit the scope thereof.

### Example 1

### Cloning of novel serine protease mBSSP4 gene

The cloning was carried out by PCR using a human brain cDNA library (Clontech) as a template and nucleotide sequences corresponding to an amino acid sequence common to serine proteases represented by
Primer 1: GTG CTC ACN GCN GCB CAY TG (SEQ ID NO: 30)
Primer 2: CCV CTR WSD CCN CCN GGC GA (SEQ ID NO: 31)
as primers. Namely, 5 µl of the template, 5 µl of 10 x ExTaq buffer, 5 µl of dNTP, 10 pmol of each of the above primers and 0.5 µl of ExTaq (TAKARA) were added and the total volume was adjusted to 50 µl with sterilized water. PCR was carried out by repeating a cycle of heating at 94°C for 0.5 minute, at 55°C for 0.5 minute and then at 72°C for 1 minutes, 35 times. The PCR product was mixed with pCR II-TOPO vector attached to TOPO TA cloning kit (Invitrogen) and the mixture was allowed to stand at room temperature for 5 minutes. Then, according to a conventional manner, *E. coli* Top 10 attached to the kit was transformed and applied to a LB (Amp⁺) plate (containing 100 µg/ml of ampicillin). According to a conventional manner, a plasmid was extracted from each colony obtained and its nucleotide sequence was determined by cycle sequencing method with a fluorescence sequencer (ABI). Homology of the sequence of each clone was examined by means of GenBank. Regarding an unknown sequence, i.e., BSSP4 gene, the full length cDNA was obtained by 5' RACE and 3' RACE and, according to the same manner as described above, the nucleotide sequence was determined. Namely, BSSP4 clone specific primers, GSP1 primers [hBSSP4F1 (SEQ ID NO: 32) or hBSSP4R1 (SEQ ID NO: 36)] and GSP2 primers [hBSSP4F2 (SEQ ID NO: 33) or hBSSP4R2 (SEQ ID NO: 37)] were prepared. PCR was carried out by using human brain Marathon-Ready cDNA (Clontech), AP1 primer attached to this reagent and either of the above GSP1 primers and heating at 94°C for 2 minutes once and repeating a cycle of heating at 94°C for 30 seconds, at 60°C for 30 seconds and then at 72°C for 30 seconds 35 times. Then, 5 µl of the PCR product diluted to 1/100, 5 µl of 10 x buffer, 5 µl of dNTP, 10 pmol of either of 10 µM of the above GSP2 primer, 10 pmol of AP2 primer attached to the above reagent and 0.5 unit of ExTaq were admixed and adjusted to 50 µl with sterilized water. Then, according to the same manner as the above, PCR was carried out. The PCR product was cloned by the above TOPO TA cloning kit and sequenced to obtain the upstream and downstream regions of the above clone. At this time, as for a clone which seemed not to cover the full length of a protein, the specific primers shown hereinafter were prepared based on the newly found nucleotide sequence. Further, based on this sequence, the primers capable of amplifying ORF as shown hereinafter [hBSSP4F6 (SEQ ID NO: 35) and hBSSP4R3/E (SEQ ID NO: 38) or hBSSP4R4/E (SEQ ID NO: 39)] were prepared and PCR carried out using human brain Marathon-ready cDNA as a template to confirm that these clones were identical. This was cloned into pCR II-TOPO vector attached to TOPO TA cloning kit to obtain the plasmid pCR II/hBSSP4 containing the full length cDNA clone. The nucleotide sequence of DNA contained in this plasmid is shown in SEQ ID NO: 1 and the amino acid sequence of hBSSP4 protein deduced from the nucleotide sequence is shown in SEQ ID NO: 2. Further, two different types of clones were obtained. The amino acid sequence of hBSSP4 represented by SEQ ID NO: 2 (the 1st to 268th amino acids) is hBSSP4 mature or active type protein composed of 268 amino acids. In the amino acid sequence represented by SEQ ID NO: 2, the -49th to -1st amino acids are a prepro or pro part and the -15th to -1st amino acids are a pro part and are considered to be a precursor of hBSSP4. As consensus sequences of serine proteases, there are Ala-Ala-His-Cys represented by the 39th to 42nd amino acids and Asp-Ser-Gly-Gly-Pro represented by the 192nd to 196th amino acids and there are one or more Asp's between these consensus sequences.

Further, 8 clones having different nucleotide sequences, perhaps, caused by alternative splicing wre obtained. The nucleotide sequences thereof are shown in SEQ ID NOS: 3, 5, 7, 9, 11, 13, 15 and 17. Further, the amino acid sequeces thereof deduced from these nucleotide sequences are shown in SEQ ID NOS: 4, 6, 8, 10, 12, 14, 16 and 18. As described above, in these sequences, there are those having either or bot consensus sequences of serine proteases and those having no consensus sequences of serine proteases. There is a possibility that these gene products (transcription product or translation product) have a function of control factors for serine proteases.

According to the same manner, 5' RACE and 3' RACE were carried out by using the primers as described hereinafter and mouse brain Marathon-Ready cDNA (Clontech) as a template, followed by cloning to obtain mouse homologous gene pCRII/mBSSP4. The nucleotide of DNA containing this plasmid is shown by SEQ ID NO: 19 and the amino acid sequence of mBSSP4 protein deduced from this nucleotide sequence is shown in SEQ ID NO: 20. The amino acid sequence of mBSSP4 represented by SEQ ID NO: 20 (the 1st to 259th amino acids) is mBSSP4 mature or active type protein composed of 259 amino acids. In the amino acid sequence represented by SEQ ID NO: 20, the -49th to 1st amino acids are a prepro or pro part and the -15th to -1st amino acids are a pro part and are considered to be a precursor of mBSSP4. As consensus sequences of serine proteases, there are Ala-Ala-His-Cys (the 39th to 42nd amino acids) and Asp-Ser-Gly-Gly-Pro (the 192nd to 196th amino acids) and there are one or more Asp's between the consensus sequences. human BSSP4

hBSSP4F1    Forward    AGGTTCCTATCATCGACTCG    RACE
(SEQ ID NO: 32)

hBSSP4F2 Forward TGAGGACATGCTGTGTGCCGG RACE
(SEQ ID NO: 33)

hBSSP4F3 | Forward | GTTGTGGCGGCGAGGACAG | mature
(SEQ ID NO: 34)

hBSSP4F6    Forward    GCCATGGTGGTTTCTGGAGC    FL*
(SEQ ID NO: 35)

hBSSP4R1 Reverse TATGGTTTGTTCAGGTTGTCC RACE
(SEQ ID NO: 36)

hBSSP4R2   Reverse  AGGGCAATGTCTGCACAGGC                    RACE
                                                        (SEQ ID NO: 37)

hBSSP4R3/E Reverse  CTGAATTCCTAGGAGCGCGCGGCGGCC          FL*
                                                  (SEQ ID NO: 38)

hBSSP4R4/E Reverse GAGAATTCGATATGTGGCAGGGTTACA FL*
(SEQ ID NO: 39)

mouse BSSR4
mBSSP4.1 Forward ACAAACCATCTCTGTTCTCAG RACE
(SEQ ID NO: 40)

mBSSP4F2 | Forward | GTCCCAGAAAGTAGGCATTG | RACE
(SEQ ID NO: 41)

mBSSP4F3    Forward    CTCCACCCATACCAGCAATG    FL*
(SEQ ID NO: 42)

mBSSP4F4    Forward    ATTGTGGAGGTGAGGACAG    mature
(SEQ ID NO: 43)

mBSSP4.2   Reverse   TGCAGAGTTCGGAGTCGATG   RACE
(SEQ ID NO: 44)

mBSSP4R2 Reverse ATCCAGCAGTCGGTCTTGGG RACE
(SEQ ID NO: 45)

mBSSP4R3/P Reverce ATTCTGCAGTTCCTTGTTCTCTCGCTCAGG FL*
(SEQ ID NO: 46)
*: for full length

### Example 2

Expression hBSSP4 or mBSSP4 gene in human being and mouse internal organs

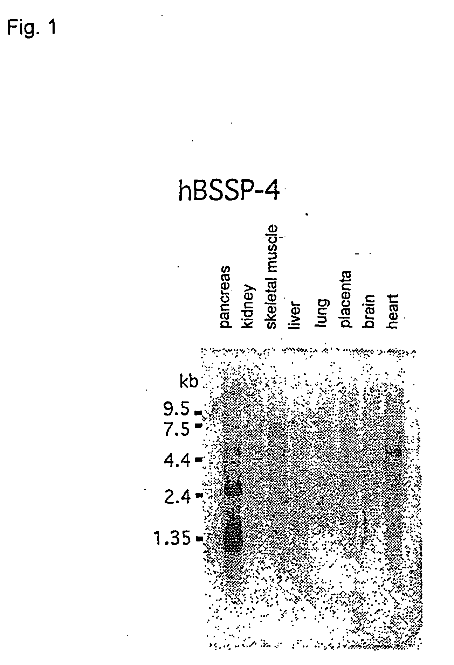

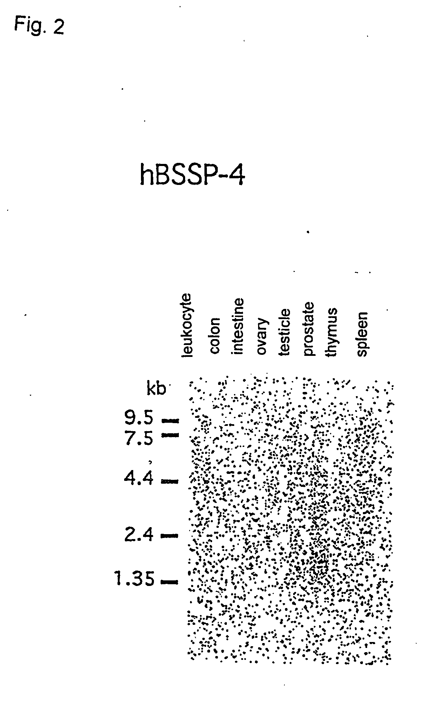

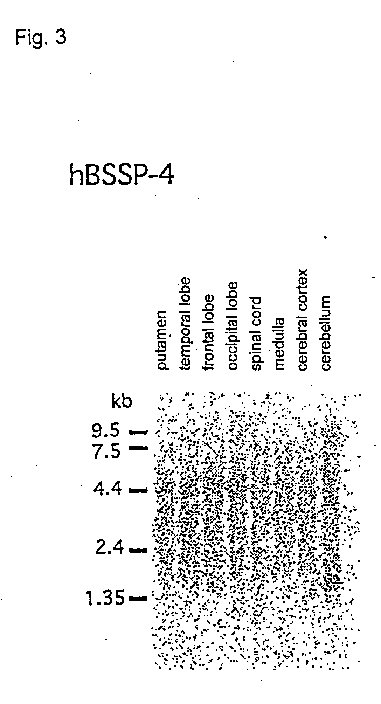

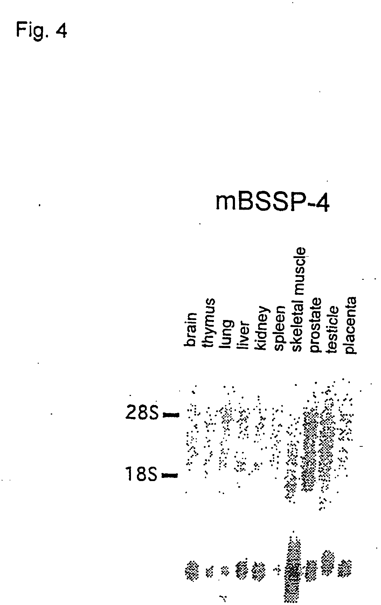

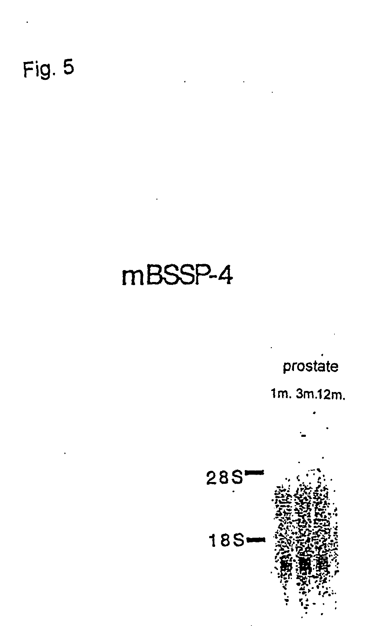

According to the protocol of QuickPrep Micro mRNA purification Kit (Amersham-Pharmacia), mRNAs were isolated from various internal organs of Balb/c mice or their fètuses. They were subjected to electrophoresis according to a conventional manner and transcribed to a nylon membrane. A probe was prepared separately by isolating a part of a nucleotide sequence encoding the mature protein of mBSSP4 from pCR II/mBSSP4, purifying it and labeling it with α-³²P dCTP. The probe was diluted with 5 x SSC and reacted with the above membrane filter at 65°C for a whole day and night. Likewise, a probe was prepared by isolating a part of a nucleotide sequence encoding the mature proteinof hBSSP4 from pCR II/hBSSP4, purifying it and labeling it with α-³²P dCTP, and diluted with 5 x SSC and the dilution was reacted with human multiple tissue blot, human multiple tissue blot II and human brain multiple blot II (Clontech) membrane. Then, the filter was washed twice each with 2 x SSC/0.1% SDS at room temperature for 30 minutes, 1 x SSC/0.1% SDS at room temperature for 30 minutes and 0.1 x SSC/0.1% SDS at 65°C for 30 minutes. The filter was exposed to an imaging plate for FLA2000 (Fuji Film) for one day to analyze the expression. The results shown in the drawings are those obtained by using human multiple tissue blot membrane (Fig. 1), human multiple tissue blot II membrane (Fig. 2), human brain multiple blot II membrane (Fig. 3) and mRNAs prepared from various internal organs of 3-month-old mice (Fig. 4) and mRNAs prepared from prostates of 1-month-old, 3-month-old and 12-month-old mice (Fig. 5). In addition, the mRNAs prepared above were subjected to RT-PCR by using Ready To Go RT-PCR Beads (Amersham-Pharmacia) and hBSSP4 and mBSSP4 gene specific primers (human being: SEQ ID NOS: 33 and 38 or 39, mouse: SEQ ID NOS: 40 and 44) according to the protocol attached to the kit.

As seen form Figs. 1 to 3, in case of northern blotting analysis, the expression of hBSSP4 was recognized in prostate (Fig. 2, the band between 1.35 to 2.4 kb) and cerebllum (Fig. 3, the band between 1.35 and 2.4 kb). The expression of mBSSP4 was recognized in prostate and skeletal muscle (Fig. 4). Further, according to the results of RT-PCR, the expression of hBSSP4 was recognized in brain, placenta, testicle and prostate of from fetuses to adults of human beings and the expression of mBSSP4 was recognized in prostate of newborns to adults of mice. Then, it is considered that the novle serine proteases of the present invention have various roles in brain, prostate, placenta, testicle and skeletal muscle. Further, the presence of the transcribed product (about 1.4 to 1.5 kb) having the nucleotide sequence of SEQ ID NO: 7 has been confirmed by the above northern blotting analysis.

### Example 3

### Expression of novel serine proteases encoded by hBSSP4 or mBSSP4 gene

### (1) Construction of expression plasmid

A cDNA region encoding the mature form of hBSSP4 or mBSSP4 protein was amplified by PCR using the plasmid pCR II/hBSSP4 or pCR II/mBSSP4 as a template (the primers used were those having the sequences of SEQ ID NOS: 34 and 39 for a human being and those having the sequences of SEQ ID NOS: 43 and 46 for mouse). The PCR product was ligated to pTrc-HisB (Invitrogen) which had been digested with BamHI and blunted with mung bean nuclease. *E. coli* JM109 was transformed by the resultant and colonies formed were analyzed by PCR to obtain *E*. *coli* containing the desired serine protease expressing plasmid pTrcHis/hBSSP4 or pTrcHis/mBSSP4.

The resultant *E*. *coli* strains were designated E. coli pTrcHis/hBSSP4 and E. coli pTrcHis/mBSSP4, respectively, and deposited at National Institute of Bioscience and Human-Technology (NIBH), Agency of Industrial Science & Technology of 1-1-3 Higashi, Tsukuba-shi, Ibaraki-ken, Japan on October 29, 1998 under the accession numbers of FERM P-17037 and FERM P-17034, respectively.

### (2) Expression of protein by E. coli containing expression plasmid

A single colony of *E. coli* having the expression plasmid was inoculated in 10 ml of LB (Amp⁺) culture medium and incubated at 37°C overnight. This was inoculated in 250 ml of LB (Amp⁺) culture medium and incubated at 37°C. When the absorbance at 600 nm became 0.5, 250 µl of 0.1 M IPTG (isopropyl-β-D-(-)-thiogalactopyranoside) was added and the incubation was continued for additional 5 hours. The *E. coli* was centrifuged and suspended in a cell disruption buffer (10 mM phosphate buffer pH 7.5, 1 mM EDTA) and sonicated on ice to disrupt *E. coli.* This was centrifuged at 14,000 r.p.m. for 20 minutes to obtain a precipitate. The precipitate was washed twice with a cell disruption buffer containing 0.5% Triton X-100™ and washed with water to remove Triton X-100™. Then, the resultant mixture was dissolved by soaking in a denaturation buffer containing 8 M urea (8M urea, 50 mM Tris pH8.5, 20 mM ME) at 37°C for 1 hour. The solution was passed through TALON metal affinity resin (Clontech), washed with the denaturation buffer containing 10 mM imidazole, and then eluted with the denaturation buffer containing 100 mM imidazole to purify the solution. The purified product was dialyzed against PBS for 3 days with exchanging the buffer every other night to obtain the protein hBSSP4-His or mBSSP4-His.

### Example 4

### Expression of novel serine protease mature protein encoded by hBSSP4 gene by using pFBTrypSigTag/hBSSP4

### (1) Construction of pFBTrypSigTag/hBSSP4

The sequences represented by SEQ ID NOS: 21 and 22 were subjected to annealing and digested with NheI and BamHI. The resultant fragment was inserted into Nhe-I-BamHI digested pSecTag2A (Invitrogen) to obtain pSecTrypHis. Twenty units of BamHI was added to 5 µg of pSecTrypHis vector and the vector was cleaved at 37°C over 4 hours. Then, 6 units of mung bean nuclease (TAKARA) was added thereto and reacted at room temperature (25°C) for 30 minutes to blunt the terminal ends. Further, the 3'-terminus side of the cloning site was cleaved with 20 units of XhoI, 1 unit of bacterial alkaline phosphatase (TAKARA) was added thereto and the reaction was carried out at 65°C for 30 minutes.

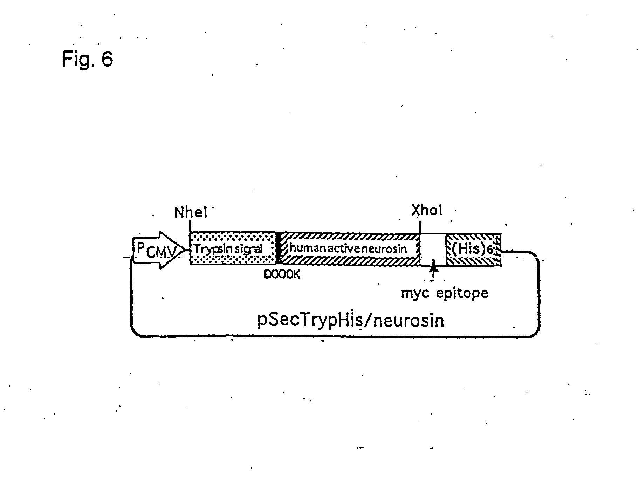

According to the same manner as that described in JP 9-149790 A or Biochim. Biophys. Acta, 1350, 11, 1997, mRNA was prepared from COLO201 cells and cDNA was synthesized to obtain the plasmid pSPORT/neurosin. cDNA of an active region of neurosin was obtained from pSPORT/neurosin by PCR using primers having the sequences represented by SEQ ID NOS: 23 and 24. Ten units of XhoI was reacted with the PCR product at 37°C for 3 hours to cleave XhoI site at the 3'-side thereof. This was inserted into pSecTrypHis by TAKARA ligation kit to obtain pSecTrypHis/neursoin (Fig. 6).

Amplification was carried out by using the primers having the sequences represented by SEQ ID NOS: 25 and 26 so that the peptide of Leu-Val-His-Gly was present at the C-terminus of the part from trypsin signal to the enterokinase recognition site of pSecTrypHis/neurosin. This was inserted between NheI and HindIII sites of pSecTag2A to construct the plasmid pTrypSig.

One µg (0.1 µl) of the plasmid pSecTab2A was treated with the restriction enzymes NheI and BamHI to completely remove a region encoding the leader sequence of IgGk. One hundred pmol portions of DANs represented by SEQ ID NOS: 47 and 48 were added to the resultant solution and the mixture was heated at 70°C for 10 minutes and subjected to annealing by allowing to stand at room temperature for 30 minutes. Two µl of I solution of DNA ligation kit Ver. 2 (TAKARA), was added to 1 µl portions of His secretory signal sequence and pSecTag2A treated by NheI and BamHI and the reaction was carried out at 16°C for 30 minutes.

To the reaction mixture was add 0.1 ml of *E. coli* competent cell XL1-Blue (STRATAGENE) and reacted on ice for 30 minutes. Then, the reaction mixture was subjected to heat shock at 42°C for 60 seconds. After standing on ice for 2 minutes, 0.9 ml of SOC culture medium (Toyo Boseki K.K.) was added thereto and the mixture was shaken with a shaker at 37°C for 1 hour. The mixture was centrifuged at 5,000 r.p.m. for 1 minutes and the supernatant was discarded. The precipitated competent cells were suspended in the liquid remained in the centrifuge tube and the suspension was applied to an ampicillin LB plates containing 100 µg/ml of ampicillin. The plates were incubated at 37°C overnight. Among the colonies formed, a colony into which DNA of His secretory signal was inserted was selected by PCR to obtain pTrypHis.

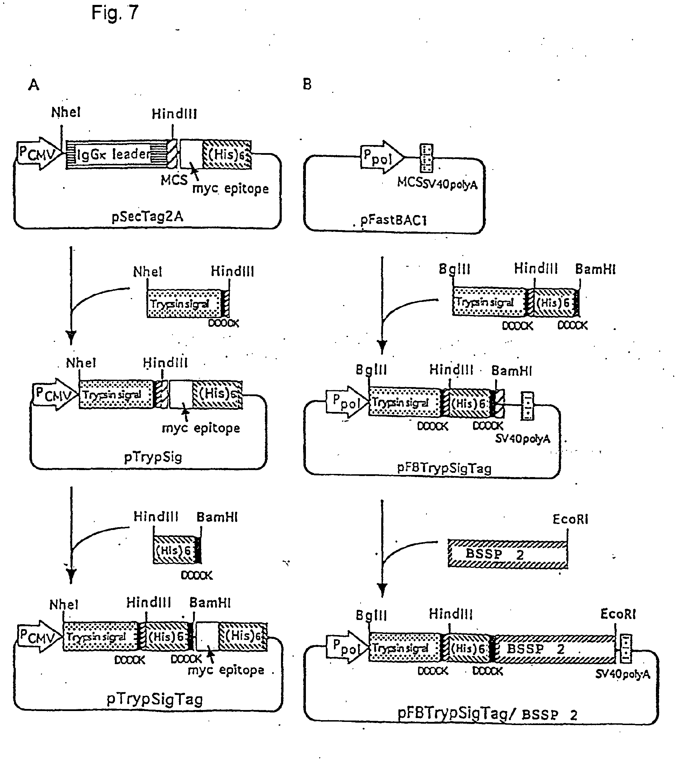

A sequence of about 200 bp containing His Tag region of pTrypHis was amplified by using primers having the sequence represented by SEQ ID NOS: 26 and 27 and a fragment of about 40 bp containing His Tag and enterokinase recognizing site formed by digestion of HindIII and BamHI was inserted into pTrypSig to construct pTrypSigTag (Fig. 7A).

cDNA was prepared by PCR of the sequence from trypsin signal to enterokinase recognizing site of pTrypSigTag using primers having the sequences represented by SEQ ID NOS: 24 and 28 and cut out by digestion with BglII and BamHI. It was inserted into BamHI site of pFastBACl (GIBCO). The insertion direction was confirmed by PCR using primers having the sequences represented by SEQ ID NOS 24 and 29. A clone into which the cDNA was inserted in the direction toward transcription and translation by polyhedrin promoter was selected to obtain pFBTrypSigTag.

Twenty units of BamHI was added to 5 µg of pFBTrypSigTag vector and the vector was cleaved at 37°C over 4 hours, followed by addition of 6 units of mung bean nuclease (TAKARA) and reaction at room temperature (25°C) for 30 minutes to blunt the terminal ends. Further, the 3'-side of the cloning site was cleaved by 20 units of EcoRI, followed by addition of 1 unit of bacterial alkaline phosphatase (TAKARA). The reaction was carried out at 65°C for 30 minutes.

cDNA of the active region of hBSSP4 was obtained by PCR according to a conventional manner using pTrcHis/hBSSP4 prepared from *E. coli.* pTrcHis/hBSSP4 (accession No. FERM P-17037) or pCRII/hBSSP4. The resultant cDNA was inserted into pFBTrypSigTag to obtain pFBTrypSigTag/hBSSP4 (Fig. 7B). At this time, correct insertion of hBSSP4 was confirmed by determining the sequence.

Bacmid DNA was transformed with pFBTrypSigTag/hBSSP4 according to a protocol of Gibco BRL BAC-TO-BAC baculovirus expression system to prepare a recombinant bacmid having chimera hBSSP4 fused with trypsinogen signal peptide, His tag and enterokinase recognizing site. When this was expressed in Sf-9 cell according to a manual of BAC-TO-BAC baculovirus expression system, it was secreted in the culture supernatant from 2 days after infection of the virus.

According to the same manner as described above, pFETrypSigTag/mBSSP4 can be prepared and secreted by using pTrcHis/mBSSP4 obtained from E. coli pTricHis/mBSSP4 (accession No. FERM P-17034) or pCRII/mBSSP4 obtained in Example 1.

### (2) Determination of enzyme activity

The recombinant fused protein hBSSP4 obtained in the culture supernatant was passed through a chelate column to purify it and, after dialysis, its enzyme activity was determined. First, the culture supernatant was applied to a chelate column (Ni-NTA-Agarose, Qiagen) with PBS buffer and eluted stepwise with a solution of imidazole (Wako Pure Chemical Industries, Ltd.) dissolved in PBS. The resultant imidazole-eluted fraction was applied to a PD-10 column (Pharmacia) to exchange to PBS buffer. Fifty µl of this sample was mixed with 10 µl of enterokinase (1 U/1 µl, Invitrogen) and the reaction was carried out at room temperature for 60 minutes. Each of various synthetic substrates (Peptide Laboratory, Boc-Gln-Ala-Arg-MCA, Boc-Phe-Ser-Arg-MCA, Bz-Arg-MCA, Boc-Val-Leu-Lys-MCA, Pyr-Gly-Arg-MCA, Pro-Phe-Arg-MCA, Boc-Val-Pro-Arg-MCA, Z-Arg-Arg-MCA, Arg-MCA, Z-Phe-Arg-MCA) was dissolved in DMSO and diluted with 1 M Tris-HCl (pH 8.0) to obtain a substrate solution. Fifty µl of 0.2 M substrate solution was added thereto and further the reaction was carried out at 37°C. After one hour, the fluorescence of AMC (7-amino-4-methylcoumalin) formed by the enzymatic reaction was measured at 380 nm of excitation wavelength and 460 nm of fluorescence wavelength to determine the activity.

As a result, the recombinant fused protein hBSSP4 has been shown to have serine protease activity. Likewise, mBSSP4 derived from a mouse showed the activity.

### INDUSTRIAL UTILITY

According to the present invention, there are provided isolated human and mouse serine protease (hBSSP4 and mBSSP4) polynucleotides, their homologous forms, mature forms, precursors and polymorphic variants. Further, according to the present invention, there are provided hBSSP4 and mBSSP4 proteins as well as compositions containing hBSSP4 and mBSSP4 polynucleotides and proteins, their production and use.

### SEQUENCE LISTING FREE TEXT

SEQ ID NO: 21: Designed oligonucleotide to construct plasmid pSecTrypHis
SEQ ID NO: 22: Designed oligonucleotide to construct plasmid pSecTrypHis
SEQ ID NO: 23: Designed oligonucleotide primer to amplify neurosin-encoding sequence
SEQ ID NO: 24: Designed oligonucleotide primer to amplify neurosin-encoding sequence
SEQ ID NO: 25: Designed oligonucleotide primer to amplify a portion of plasmid pSecTrypHis/Neurosin
SEQ ID NO: 26: Designed oligonucleotide primer to amplify a portion of plasmid pSecTrypHis/Neurosin
SEQ ID NO: 27: Designed oligonucleotide primer to amplify a portion of plasmid pTrypHis
SEQ ID NO: 28: Designed oligonucleotide primer to amplify a portion of plasmid pTrypSigTag
SEQ ID NO: 29: Designed oligonucleotide primer to amplify a portion of plasmid pFBTrypSigTag
SEQ ID NO: 30: Designed oligonucleotide primer to amplify conserved region of serin proteases-encoding sequence; n is a, c, g or t.
SEQ ID NO: 31: Designed oligonucleotide primer to amplify conserved region of serin proteases-encoding sequence; n is a, c, g or t.
SEQ ID NO: 32: Designed oligonucleotide primer designated as hBSSP4F1 for RACE for human BSSP4 (forward)
SEQ ID NO: 33: Designed oligonucleotide primer designated as hBSSP4F2 for RACE for human BSSP4 (forward)
SEQ ID NO: 34: Designed oligonucleotide primer designated as hBSSP4F3 to amplify mature human BSSP4-encoding region (forward)
SEQ ID NO: 35: Designed oligonucleotide primer designated as hBSSP4F6 to amplify full-length human BSSP4-encoding mRNA (forward)
SEQ ID NO: 36: Designed oligonucleotide primer designated as hBSSP4R1 for RACE for human BSSP4 (reverse)
SEQ ID NO: 37: Designed oligonucleotide primer designated as hBSSP4R2 for RACE for human BSSP4 (reverse)
SEQ ID NO: 38: Designed oligonucleotide primer designated as hBSSP4R3/E to amplify full-length human BSSP4-encoding mRNA (reverse)
SEQ ID NO: 39: Designed oligonucleotide primer designated as hBSSP4R4/E to amplify full-length human BSSP4-encoding mRNA (reverse)
SEQ ID NO: 40: Designed oligonucleotide primer designated as mBSSP4.1 for RACE for mouse BSSP4 (forward)
SEQ ID NO: 41: Designed oligonucleotide primer designated as mBSSP4F2 for RACE for mouse BSSP4 (forward)
SEQ ID NO: 42: Designed oligonucleotide primer designated as mBSSP4F3 to amplify full-length mouse BSSP4-encoding mRNA (forward)
SEQ ID NO: 43: Designed oligonucleotide primer designated as mBSSP4F4 to amplify mature mouse BSSP4-encoding region (forward)
SEQ ID NO: 44: Designed oligonucleotide primer designated as mBSSP4.2 for RACE for mouse BSSP4 (reverse)
SEQ ID NO: 45: Designed oligonucleotide primer designated as mBSSP4R2 for RACE for mouse BSSP4 (reverse)
SEQ ID NO: 46: Designed oligonucleotide primer designated as mBSSP4R3/P to amplify full-length mouse BSSP4-encoding mRNA (reverse)
SEQ ID NO: 47: Designed oligonucleotide to construct plasmid pTrypHis
SEQ ID NO: 48: Designed oligonucleotide to construct plasmid pTrypHis

## Claims

1. A protein having the amino acid sequence composed of 268 amino acids represented by the 1st to 268th amino acids of SEQ ID NO: 2; or a protein having an amino acid sequence derived from the amino acid sequence represented by SEQ ID NO: 2 by deletion, substitution or addition of one to several amino acids and having the same property as that of the protein having the amino acid sequence represented by the 1st to 268th amino acids of SEQ ID NO: 2; or a modified derivative thereof.

2. A nucleotide sequence represented by the 151st to 954th bases of SEQ ID NO: 1; a nucleotide sequence encoding the amino acid sequence represented by the 1st to 268th amino acids of SEQ ID NO: 2; or a nucleotide sequence hybridizable with a nucleotide sequence which is complementary to the above nucleotide sequence under stringent conditions and encoding a protein having the same property as that of the protein having the amino acid sequence represented by the 1st to 268th amino acids of SEQ ID NO: 2.

3. A protein having the amino acid sequence composed of 270 amino acids represented by the 1st to 270th amino acids of SEQ ID NO: 4; or a protein having an amino acid sequence derived from the amino acid sequence represented by the 1st to 270th amino acids of SEQ ID NO: 4 by deletion, substitution or addition of one to several amino acids and having the same property as that of the protein having the amino acid sequence represented by the 1st to 270th amino acids of SEQ ID NO: 4; or a modified derivative thereof.

4. A nucleotide sequence represented by the 151st to 960th bases of SEQ ID NO: 3; a nucleotide sequence encoding the amino acid sequence represented by the 1st to 270th amino acids of SEQ ID NO: 4; or a nucleotide sequence hybridizable with a nucleotide sequence which is complementary to the above nucleotide sequence under stringent conditions and encoding a protein having the same property as that of the protein having the amino acid sequence represented by the 1st to 270th amino acids of SEQ ID NO: 4.

5. A protein having the amino acid sequence composed of 257 amino acids represented by the 1st to 257th amino acids of SEQ ID NO: 6; or a protein having an amino acid sequence derived from the amino acid sequence represented by the 1st to 257th amino acids of SEQ ID NO: 6 by deletion, substitution or addition of one to several amino acids and having the same property as that of the protein having the amino acid sequence represented by the 1st to 257th amino acids of SEQ ID NO: 6; or a modified derivative thereof.

6. A nucleotide sequence represented by the 151st to 921st bases of SEQ ID NO: 5; a nucleotide sequence encoding the amino acid sequence represented by the 1st to 257th amino acids of SEQ ID NO: 6; or a nucleotide sequence hybridizable with a nucleotide sequence which is complementary to the above nucleotide sequence under stringent conditions and encoding a protein having the same property as that of the protein having the amino acid sequence represented by the 1st to 257th amino acids of SEQ ID NO: 6.

7. A protein having the amino acid sequence composed of 97 amino acids represented by the 1st to 97th amino acids of SEQ ID NO: 8; or a protein having an amino acid sequence derived from the amino acid sequence represented by the 1st to 97th amino acids of SEQ ID NO: 8 by deletion, substitution or addition of one to several amino acids and having the same property as that of the protein having the amino acid sequence represented by the 1st to 97th amino acids of SEQ ID NO: 8; or a modified derivative thereof.

8. A nucleotide sequence represented by the 151st to 441st bases of SEQ ID NO: 7; a nucleotide sequence encoding the amino acid sequence represented by the 1st to 97th amino acids of SEQ ID NO: 8; or a nucleotide sequence hybridizable with a nucleotide sequence which is complementary to the above nucleotide sequence under stringent conditions and encoding a protein having the same property as that of the protein having the amino acid sequence represeneted by the 1st to 97th amino acids of SEQ ID NO: 8.

9. A protein having the amino acid sequence composed of 158 amino acids represented by the 1st to 158th amino acids of SEQ ID NO: 10; or a protein having an amino acid sequence derived from the amino acid sequence represented by the 1st to 158th amino acids of SEQ ID NO: 10 by deletion, substitution or addition of one to several amino acids and having the same property as that of the protein having the amino acid sequence represented by the 1st to 158th amino acids of SEQ ID NO: 10; or a modified derivative thereof.

10. A nucleotide sequence represented by the 151st to 624th bases of SEQ ID NO: 9; a nucleotide sequence encoding the amino acid sequence represented by the 1st to 158th amino acids of SEQ ID NO: 10; or a nucleotide sequence hybridizable with a nucleotide sequence which is complementary to the above nucleotide sequence under stringent conditions and encoding a protein having the same property as that of the protein having the amino acid sequence represented by the 1st to 158th amino acids of SEQ ID NO: 10.

11. A protein having the amino acid sequence composed of 82 amino acids represented by the 1st to 82nd amino acids of SEQ ID NO: 12; or a protein having an amino acid sequence derived from the amino acid sequence represented by the 1st to 82nd amino acids of SEQ ID NO: 12 by deletion, substitution or addition of one to several amino acids and having the same property as that of the protein having the amino acid sequence represented by the 1st to 82nd amino acids of SEQ ID NO: 12; or a modified derivative thereof.

12. A nucleotide sequence represented by the 151th to 396th bases of SEQ ID NO: 11; a nucleotide sequence encoding the amino acid sequence represented by the 1st to 82nd amino acids of SEQ ID NO: 12; or a nucleotide sequence hybridizable with a nucleotide sequence which is complementary to the above nucleotide sequence under stringent conditions and encoding a protein having the same property as that of the protein having the amino acid sequence represented by the 1st to 82th amino acids of SEQ ID NO: 12.

13. A protein having the amino acid sequence composed of 185 amino acids represented by the 1st to 185th amino acids of SEQ ID NO: 14; or a protein having an amino acid sequence derived from the amino acid sequence represented by the 1st to 185th amino acids of SEQ ID NO: 14 by deletion, substitution or addition of one to several amino acids and having the same property as that of the protein having the amino acid sequence represented by the 1st to 185th amino acids of SEQ ID NO: 14; or a modified derivative thereof.

14. A nucleotide sequence represented by the 151st to 705th bases of SEQ ID NO: 13; a nucleotide sequence encoding the amino acid sequence represented by the 1st to 185th amino acids of SEQ ID NO: 14; or a nucleotide sequence hybridizable with a nucleotide sequence which is complementary to the above nucleotide sequence under stringent conditions and encoding a protein having the same property as that of the protein having the amino acid sequence represented by the 1st to 185th amino acids of SEQ ID NO: 14.

15. A protein having the amino acid sequence composed of 80 amino acids represented by the 1st to 80th amino acids of SEQ ID NO: 16; or a protein having an amino acid sequence derived from the amino acid sequence represented by the 1st to 80th amino acids of SEQ ID NO: 16 by deletion, substitution or addition of one to several amino acids and having the same property as that of the protein having the amino acid sequence represented by the 1st to 80th amino acids of SEQ ID NO: 16; or a modified derivative thereof.

16. A nucleotide sequence represented by the 151st to 390th bases of SEQ ID NO: 15; a nucleotide sequence encoding the amino acid sequence represented by the 1st to 80th amino acids of SEQ ID NO: 16; or a nucleotide sequence hybridizable with a nucleotide sequence which is complementary to the above nucleotide sequence under stringent conditions and encoding a protein having the same property as that of the protein having the amino acid sequence represented by the 1st to 80th amino acids of SEQ ID NO: 16.

17. A protein having the amino acid sequence composed of 253 amino acids represented by the 1st to 253rd amino acids of SEQ ID NO: 18; or a protein having an amino acid sequence derived from the amino acid sequence represented by the 1st to 253rd amino acids of SEQ ID NO: 18 by deletion, substitution or addition of one to several amino acids and having the same property as that of the protein having the amino acid sequence represented by the 1st to 253rd amino acids of SEQ ID NO: 18; or a modified derivative thereof.

18. A nucleotide sequence represented by the 151st to 909th bases of SEQ ID NO: 17; a nucleotide sequence encoding the amino acid sequence represented by the 1st to 253rd amino acids of SEQ ID NO: 18; or a nucleotide sequence hybridizable with a nucleotide sequence which is complementary to the above nucleotide sequence under stringent conditions and encoding a protein having the same property as that of the protein having the amino acid sequence represented by the 1st to 253rd amino acids of SEQ ID NO: 18.

19. A protein having the amino acid sequence composed of 34 amino acids represented by the -49th to -16th amino acids of SEQ ID NO: 2; or a protein having an amino acid sequence derived from the amino acid sequence represented by the -49th to -16th amino acids of SEQ ID NO: 2 by deletion, substitution or addition of one to several amino acids and having the same property as that of the protein having the amino acid sequence represented by the -49th to -16th amino acids of SEQ ID NO: 2; or a modified derivative or fragment thereof.

20. A nucleotide sequence represented by the 4th to 105th bases of SEQ ID NO: 1; a nucleotide sequence encoding the amino acid sequence represented by the -49th to -16th amino acids of SEQ ID NO: 2; or a nucleotide sequence hybridizable with a nucleotide sequence which is complementary to the above nucleotide sequence under stringent conditions and encoding a protein having the same property as that of the protein having the amino acid sequence represented by the -49th to -16th amino acids of SEQ ID NO: 2; or a fragment thereof.

21. A protein having the amino acid sequence composed of 15 amino acids represented by the -15th to -1st amino acids of SEQ ID NO: 2; or a protein having an amino acid sequence derived from the amino acid sequence represented by the -15th to -1st amino acids of SEQ ID NO: 2 by deletion, substitution or addition of one to several amino acids and having the same property as that of the protein having the amino acid sequence represented by the -15th to -1st amino acids of SEQ ID NO: 2; or a modified derivative or fragment thereof.

22. A nucleotide sequence represented by the 106th to 150th bases of SEQ ID NO: 1; a nucleotide sequence encoding the amino acid sequence represented by the -15th to -1st amino acids of SEQ ID NO: 2; or a nucleotide sequence hybridizable with a nucleotide sequence which is complementary to the above nucleotide sequence under stringent conditions and encoding a protein having the same property as that of the protein having the amino acid sequence represented by the -15th to -1st amino acids of SEQ ID NO: 2; or a fragment thereof.

23. A protein having the amino acid sequence composed of 259 amino acids represented by the 1st to 259th amino acids of SEQ ID NO: 20; or a protein having an amino acid sequence derived from the amino acid sequence represented by the 1st to 259th amino acids of SEQ ID NO: 20 by deletion, substitution or addition of one to several amino acids and having the same property as that of the protein having the amino acid sequence represented by the 1st to 259th amino acids of SEQ ID NO: 20; or a modified derivative thereof.

24. A nucleotide sequence represented by the 227th to 1003rd bases of SEQ ID NO: 19; a nucleotide sequence encoding the amino acid sequence represented by the 1st to 259th amino acids of SEQ ID NO: 20; or a nucleotide sequence hybridizable with a nucleotide sequence which is complementary to the above nucleotide sequence under stringent conditions and encoding a protein having the same property as that of the protein having the amino acid sequence represented by the 1st to 259th amino acids of SEQ ID NO: 20.

25. A protein having the amino acid sequence composed of 34 amino acids represented by the -49th to -16th amino acids of SEQ ID NO: 20; or a protein having an amino acid sequence derived from the amino acid sequence represented by the -49th to -16th amino acids of SEQ ID NO: 20 by deletion, substitution or addition of one to several amino acids and having the same property as that of the protein having the amino acid sequence represented by the -49th to -16th amino acids of SEQ ID NO: 20; or a modified derivative or fragment thereof.

26. A nucleotide sequence represented by the 80th to 181st bases of SEQ ID NO: 19; a nucleotide sequence encoding the amino acid sequence represented by the -49th to -16th amino acids of SEQ ID NO: 20; or a nucleotide sequence hybridizable with a nucleotide sequence which is complementary to the above nucleotide sequence under stringent conditions and encoding a protein having the same property as that of the protein having the amino acid sequence represented by the -49th to -16th amino acids of SEQ ID NO: 20; or a fragment thereof.

27. A protein having the amino acid sequence composed of 15 amino acids represented by the -15th to -1st amino acids of SEQ ID NO: 20; or a protein having an amino acid sequence derived from the amino acid sequence represented by the -15th to -1st amino acids of SEQ ID NO: 20 by deletion, substitution or addition of one to several amino acids and having the same property as that of the protein having the amino acid sequence represented by the -15th to -1st amino acids of SEQ ID NO: 20; or a modified derivative or fragment thereof.

28. A nucleotide sequence represented by the 182th to 226th bases of SEQ ID NO: 19; a nucleotide sequence encoding the amino acid sequence represented by the -15th to -1st amino acids of SEQ ID NO: 20; or a nucleotide sequence hybridizable with a nucleotide sequence which is complementary to the above nucleotide sequence under stringent conditions and encoding a protein having the same property as that of the protein having the amino acid sequence represented by the -15th to -1st amino acids of SEQ ID NO: 20; or a fragment thereof.

29. A protein having the amino acid sequence composed of 317 amino acids represented by the -49th to 268th amino acids of SEQ ID NO: 2; or a protein having an amino acid sequence derived from the amino acid sequence represented by the -49th to 268th amino acids of SEQ ID NO: 2 by deletion, substitution or addition of one to several amino acids and having the same property as that of the protein having the amino acid sequence represented by the -49th to 268th amino acids of SEQ ID NO: 2; or a modified derivative thereof.

30. A nucleotide sequence represented by the 4th to 954th bases of SEQ ID NO: 1; a nucleotide sequence encoding the amino acid sequence represented by the -49th to 268th amino acids of SEQ ID NO: 2; or a nucleotide sequence hybridizable with a nucleotide sequence which is complementary to the above nucleotide sequence under stringent conditions and encoding a protein having the same property as that of the protein having the amino acid sequence represented by the -49th to 268th amino acids of SEQ ID NO: 2.

31. A protein having the amino acid sequence composed of 283 amino acids represented by the -15th to 268th amino acids of SEQ ID NO: 2; or a protein having an amino acid sequence derived from the amino acid sequence represented by the -15th to 268th amino acids of SEQ ID NO: 2 by deletion, substitution or addition of one to several amino acids and having the same property as that of the protein having the amino acid sequence represented by the -15th to 268th amino acids of SEQ ID NO: 2; or a modified derivative thereof.

32. A nucleotide sequence represented by the 106th to 954th bases of SEQ ID NO: 1; a nucleotide sequence encoding the amino acid sequence represented by the -15th to 268th amino acids of SEQ ID NO: 2; or a nucleotide sequence hybridizable with a nucleotide sequence which is complementary to the above nucleotide sequence under stringent conditions and encoding a protein having the same property as that of the protein having the amino acid sequence represented by the -15th to 268th amino acids of SEQ ID NO: 2.

33. A protein having the amino acid sequence composed of 319 amino acids represented by the -49th to 270th amino acids of SEQ ID NO: 4; or a protein having an amino acid sequence derived from the amino acid sequence represented by the -49th to 270th amino acids of SEQ ID NO: 4 by deletion, substitution or addition of one to several amino acids and having the same property as that of the protein having the amino acid sequence represented by the -49th to 270th amino acids of SEQ ID NO: 4; or a modified derivative thereof.

34. A nucleotide sequence represented by the 4th to 960th bases of SEQ ID NO: 3; a nucleotide sequence encoding the amino acid sequence represented by the -49th to 270th amino acids of SEQ ID NO: 4; or a nucleotide sequence hybridizable with a nucleotide sequence which is complementary to the above nucleotide sequence under stringent conditions and encoding a protein having the same property as that of the protein having the amino acid sequence represented by the -49th to 270th amino acids of SEQ ID NO: 4.

35. A protein having the amino acid sequence composed of 285 amino acids represented by the -15th to 270th amino acids of SEQ ID NO: 4; or a protein having an amino acid sequence derived from the amino acid sequence represented by the -15th to 270th amino acids of SEQ ID NO: 4 by deletion, substitution or addition of one to several amino acids and having the same property as that of the protein having the amino acid sequence represented by the -15th to 270th amino acids of SEQ ID NO: 4; or a modified derivative thereof.

36. A nucleotide sequence represented by the 106th to 960th bases of SEQ ID NO: 3; a nucleotide sequence encoding the amino acid sequence represented by the -15th to 270th amino acids of SEQ ID NO: 4; or a nucleotide sequence hybridizable with a nucleotide sequence which is complementary to the above nucleotide sequence under stringent conditions and encoding a protein having the same property as that of the protein having the amino acid sequence represented by the -15th to 270th amino acids of SEQ ID NO: 4.

37. A protein having the amino acid sequence composed of 306 amino acids represented by the -49th to 257th amino acids of SEQ ID NO: 6; or a protein having an amino acid sequence derived from the amino acid sequence represented by the -49th to 257th amino acids of SEQ ID NO: 6 by deletion, substitution or addition of one to several amino acids and having the same property as that of the protein having the amino acid sequence represented by the -49th to 257th amino acids of SEQ ID NO: 6; or a modified derivative thereof.

38. A nucleotide sequence represented by the 4th to 921th bases of SEQ ID NO: 5; a nucleotide sequence encoding the amino acid sequence represented by the -49th to 257th amino acids of SEQ ID NO: 6; or a nucleotide sequence hybridizable with a nucleotide sequence which is complementary to the above nucleotide sequence under stringent conditions and encoding a protein having the same property as that of the protein having the amino acid sequence represented by the -49th to 257th amino acids of SEQ ID NO: 6.

39. A protein having the amino acid sequence composed of 272 amino acids represented by the -15th to 257th amino acids of SEQ ID NO:. 6; or a protein having an amino acid sequence derived from the amino acid sequence represented by the -15th to 257th amino acids of SEQ ID NO: 6 by deletion, substitution or addition of one to several amino acids and having the same property as that of the protein having the amino acid sequence represented by the -15th to 257th amino acids of SEQ ID NO: 6; or a modified derivative thereof.

40. A nucleotide sequence represented by the 106th to 921th bases of SEQ ID NO: 5; a nucleotide sequence encoding the amino acid sequence represented by the -15th to 257th amino acids of SEQ ID NO: 6; or a nucleotide sequence hybridizable with a nucleotide sequence which is complementary to the above nucleotide sequence under stringent conditions and encoding a protein having the same property as that of the protein having the amino acid sequence represented by the -15th to 257th amino acids of SEQ ID NO: 6.

41. A protein having the amino acid sequence composed of 308 amino acids represented by the -49th to 259th amino acids of SEQ ID NO: 20; or a protein having an amino acid sequence derived from the amino acid sequence represented by the -49th to 259th amino acids of SEQ ID NO: 20 by deletion, substitution or addition of one to several amino acids and having the same property as that of the protein having the amino acid sequence represented by the -49th to 259th amino acids of SEQ ID NO: 20; or a modified derivative thereof.

42. A nucleotide sequence represented by the 80th to 1003rd bases of SEQ ID NO: 19; a nucleotide sequence encoding the amino acid sequence represented by the -49th to 259th amino acids of SEQ ID NO: 20; or a nucleotide sequence hybridizable with a nucleotide sequence which is complementary to the above nucleotide sequence under stringent conditions and encoding a protein having the same property as that of the protein having the amino acid sequence represented by the -49th to 259th amino acids of SEQ ID NO: 20.

43. A protein having the amino acid sequence composed of 274 amino acids represented by the -15th to 259th amino acids of SEQ ID NO: 20; or a protein having an amino acid sequence derived from the amino acid sequence represented by the -15th to 259th amino acids of SEQ ID NO: 20 by deletion, substitution or addition of one to several amino acids and having the same property as that of the protein having the amino acid sequence represented by the -15th to 259th amino acids of SEQ ID NO: 20; or a modified derivative thereof.

44. A nucleotide sequence represented by the 182nd to 1003rd bases of SEQ ID NO: 19; a nucleotide sequence encoding the amino acid sequence represented by the -15th to 259th amino acids of SEQ ID NO: 20; or a nucleotide sequence hybridizable with a nucleotide sequence which is complementary to the above nucleotide sequence under stringent conditions and encoding a protein having the same property as that of the protein having the amino acid sequence represented by the -15th to 259th amino acids of SEQ ID NO: 20.

45. A nucleotide sequence represented by SEQ ID NO: 1; or a nucleotide sequence hybridizable with a nucleotide sequence which is complementary to the above nucleotide sequence under stringent conditions and encoding a protein having the same property as that of the protein encoded by the nucleotide sequnece represented by SEQ ID NO: 1.

46. A nucleotide sequence represented by SEQ ID NO: 3; or a nucleotide sequence hybridizable with a nucleotide sequence which is complementary to the above nucleotide sequence under stringent conditions and encoding a protein having the same property as that of the protein encoded by the nucleotide sequnece represented by SEQ ID NO: 3.

47. A nucleotide sequence represented by SEQ ID NO: 5; or a nucleotide sequence hybridizable with a nucleotide sequence which is complementary to the above nucleotide sequence under stringent conditions and encoding a protein having the same property as that of the protein encoded by the nucleotide sequnece represented by SEQ ID NO: 5.

48. A nucleotide sequence represented by SEQ ID NO: 7; or a nucleotide sequence hybridizable with a nucleotide sequence which is complementary to the above nucleotide sequence under stringent conditions and encoding a protein having the same property as that of the protein encoded by the nucleotide sequnece represented by SEQ ID NO:7.

49. A nucleotide sequence represented by SEQ ID NO: 9; or a nucleotide sequence hybridizable with a nucleotide sequence which is complementary to the above nucleotide sequence under stringent conditions and encoding a protein having the same property as that of the protein encoded by the nucleotide sequnece represented by SEQ ID NO: 9.

50. A nucleotide sequence represented by SEQ ID NO: 11; or a nucleotide sequence hybridizable with a nucleotide sequence which is complementary to the above nucleotide sequence under stringent conditions and encoding a protein having the same property as that of the protein encoded by the nucleotide sequnece represented by SEQ ID NO: 11.

51. A nucleotide sequence represented by SEQ ID NO: 13; or a nucleotide sequence hybridizable with a nucleotide sequence which is complementary to the above nucleotide sequence under stringent conditions and encoding a protein having the same property as that of the protein encoded by the nucleotide sequnece represented by SEQ ID NO: 13.

52. A nucleotide sequence represented by SEQ ID NO: 15; or a nucleotide sequence hybridizable with a nucleotide sequence which is complementary to the above nucleotide sequence under stringent conditions and encoding a protein having the same property as that of the protein encoded by the nucleotide sequnece represented by SEQ ID NO: 15.

53. A nucleotide sequence represented by SEQ ID NO: 17; or a nucleotide sequence hybridizable with a nucleotide sequence which is complementary to the above nucleotide sequence under stringent conditions and encoding a protein having the same property as that of the protein encoded by the nucleotide sequnece represented by SEQ ID NO: 17.

54. A nucleotide sequence represented by SEQ ID NO: 19; or a nucleotide sequence hybridizable with a nucleotide sequence which is complementary to the above nucleotide sequence under stringent conditions and encoding a protein having the same property as that of the protein encoded by the nucleotide sequnece represented by SEQ ID NO: 19.

55. A vector comprising the nucleotide sequence according to any one of claims 2, 4, 6, 8, 10, 12, 14, 16, 18, 20, 22, 24, 26, 28, 30, 32, 34, 36, 38, 40, 42 and 44-54.

56. Transformed cells having the nucleotide sequence according to any one of claims 2, 4, 6, 8, 10, 12, 14, 16, 18, 20, 22, 24, 26, 28, 30, 32, 34, 36, 38, 40, 42 and 44-54 in an expressible state.

57. A process for producing a protein which comprises culturing cells transformed with the nucleotide sequence according to any one of claims 2, 4, 6, 8, 10, 12, 14, 16, 18, 20, 22, 30, 32, 34, 36, 38, 40 and 45-53, and collecting hBSSP4 produced.

58. A process for producing a protein which comprises culturing cells transformed with the nucleotide sequence according to any one of claims 24, 26, 28, 42, 44 or 54, and collecting mBSSP4 produced.

59. The process according to claim 57 or 58, wherein the cells are *E. coli* cells, animal cells or insect cells.

60. A non-human transgenic animal whose expression level of BSSP4 gene has been altered.

61. The non-human transgenic animal according to claim 60, wherein BSSP4 gene is cDNA, genomic DNA or synthetic DNA encoding BSSP4.

62. The non-human transgenic animal according to claim 60, wherein the expression level has been altered by mutating a gene expression regulatory site.

63. A knockout mouse whose mBSSP4 gene function is deficient.

64. An antibody against the protein according to any one of claims 1, 3, 5, 7, 9, 11, 13, 15, 17, 19, 21, 23, 25, 27, 29, 31, 33, 35, 37, 39, 41 and 43 or a fragment thereof.

65. The antibody according to claim 64 which is a polyclonal antibody, a monoclonal antibody or a peptide antibody.

66. A process for producing a monoclonal antibody against the protein according to any one of claims 1, 3, 5, 7, 9, 11, 13, 15, 17, 19, 21, 23, 25, 27, 29, 31, 33, 35, 37, 39, 41 and 43 or a fragment thereof which comprises administering the protein according to any one of claims 1, 3, 5, 7, 9, 11, 13, 15, 17, 19, 21, 23, 25, 27, 29, 31, 33, 35, 37, 39, 41 and 43 or a fragment thereof to a warm-blooded animal other than a human being, selecting the animal whose antibody titer is recognized, collecting its spleen or lymph node, fusing the antibody producing cells contained therein with myeloma cells to prepare a monoclonal antibody producing hybridoma.

67. A method for determining the protein according to any one of claims 1, 3, 5, 7, 9, 11, 13, 15, 17, 19, 21, 23, 25, 27, 29, 31, 33, 35, 37, 39, 41 and 43 or a fragment thereof in a specimen which is based on immunological binding of an antibody against the protein or a fragment thereof to the protein or a fragment thereof.

68. A method for determining hBSSP4 or a fragment thereof in a specimen which comprises reacting a monoclonal antibody or 'a polyclonal antibody against the protein according to any one of claims 1, 3, 5, 7, 9, 11, 13, 15, 17, 19, 21, 29, 31, 33, 35, 37 and 39 or a fragment thereof and a labeled antibody with hBSSP4 or a fragment thereof in the specimen to detect a sandwich complex produced.

69. A method for determining hBSSP4 or a fragment thereof in a specimen which comprises reacting a monoclonal antibody or a polyclonal antibody against the protein according to any one of claims 1, 3, 5, 7, 9, 11, 13, 15, 17, 19, 21, 29, 31, 33, 35, 37 and 39 or a fragment thereof with labeled hBBSP4 and hBSSP4 or a fragment thereof in the specimen competitively to detect an amount of hBSSP4 or a fragment thereof in the specimen based on an amount of the labeled hBBSP4 reacted with the antibody.

70. The method according to any one of claims 67-69, wherein the specimen is a body fluid.

71. A diagnostic marker for diseases in tissues comprising the protein according to any one of claims 1, 3, 5, 7, 9, 11, 13, 15, 17, 19, 21, 23, 25, 27, 29, 31, 33, 35, 37, 39, 41 and 43.

72. The marker according to claim 71 to be used for diagnosis of Alzheimer's disease or epilepsy in brain.

73. The marker according to claim 71 to be used for diagnosis of cancer or inflammation of brain, prostate or testicle.

74. The marker according to claim 71 to be used for diagnosis of sterility in semen or sperms

75. The marker according to claim 71 to be used for diagnosis of prostatic hypertrophy in prostate.
